(19) Europäisches Patentamt
European Patent Office
Office européen des brevets

(11) EP 1 842 925 B1

(12) **EUROPEAN PATENT SPECIFICATION**

(45) Date of publication and mention of the grant of the patent:
**17.02.2010 Bulletin 2010/07**

(51) Int Cl.:
***C12Q 1/68*** *(2006.01)*

(21) Application number: **07014693.1**

(22) Date of filing: **01.09.2005**

(54) **Method for quantifying initial concentration of nucleic acids from real-time amplification data**

Verfahren zur Bestimmung einer anfänglichen Nukleinsäurekonzentration anhand von real-time Amplifikationsdaten

Procédé pour la quantification de la concentration initiale d'acide nucléique à partir de données d'amplification d'acide nucléique en temps réel

(84) Designated Contracting States:
**DE FR GB**

(30) Priority: **01.09.2004 KR 20040069560**
**13.04.2005 KR 20050030745**

(43) Date of publication of application:
**10.10.2007 Bulletin 2007/41**

(62) Document number(s) of the earlier application(s) in accordance with Art. 76 EPC:
**05019029.7 / 1 632 580**

(73) Proprietor: **Samsung Electronics Co., Ltd.**
**Suwon-si, Gyeonggi-Do (KR)**

(72) Inventors:
- **Namkoong, Kak**
**Songpa-gu**
**Seoul (KR)**
- **Kim, Jin-Tae**
**Hwaseong-si**
**Gyeonggi-do (KR)**
- **Lee, Young-sun**
**Seongnam-si**
**Gyeonggi-do (KR)**
- **Kim, Young-a**
**Suwon-si**
**Gyeonggi-do (KR)**

(74) Representative: **Grünecker, Kinkeldey,**
**Stockmair & Schwanhäusser**
**Anwaltssozietät**
**Leopoldstrasse 4**
**80802 München (DE)**

(56) References cited:
**US-A1- 2002 028 452** **US-A1- 2003 165 832**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 842 925 B1

## Description

The present invention relates to a method for quantifying the initial nucleic acid concentration, and more particularly, to a method for quantifying the initial nucleic acid concentration from real-time nucleic acid amplification data, which is obtained by polymerase chain reaction (PCR), ligase chain reaction (LCR), strand displacement amplification (SDA), nucleic acid sequence-based amplification (NASBA), transcription mediated amplification (TMA), rolling-circle amplification (RCA), and so on.

Polymerase chain reaction (PCR) is most widely used among a variety of analysis methods for detecting and quantifying nucleic acid. A principle of the PCR is disclosed in U.S. Patent Nos. 4,683,195 and 4,683,202.

Conventional PCR gives only a qualitative result of an amplified DNA at end point by an agarose gel. However, such conventional PCR has a problem in that it cannot be used in quantitative analyses. In order to solve the problem, real-time PCR has been developed. The real-time PCR uses an optical detection system to detect in real time the fluorescence intensity that is proportional to the concentration of an amplified DNA, such that a quantitative analysis of DNA is possible.

Conventional methods for quantifying the initial concentration of a nucleic acid from a nucleic acid amplification data are disclosed in U.S. Patent Nos. 6,303,305 and 6,503,720. In these conventional methods, a nucleic acid is amplified and a function representing the amount of the nucleic acid in each amplification cycle is obtained. Then, n-th order derivative of the function is calculated and the initial concentration of the nucleic acid is calculated from the result. Also, a quantitative analysis method using the maximum value of the derivative as threshold cycle (Ct) is disclosed in U.S. Patent No. 6,303,305, and a quantitative analysis method using maximum, minimum and zero value of the derivative as Ct is disclosed in U.S. Patent No. 6,503,720.

US 2003/0165832 refers to a method for the quantification of a target nucleic acid in a sample comprising the following steps: (i) determination of the amplification efficiency of the target nucleic acid under defined amplification conditions, (ii) amplification of the target nucleic acid contained in the sample under the same defined reaction conditions, (iii) measuring the amplification in real-time, (iv) quantification of the original amount of target nucleic acid in the sample by correction of the original amount derived from step (iii) with the aid of the determined amplification efficiency. The efficiency correction of PCR reactions for the quantification of nucleic acids can be used for absolute quantification with the aid of an external or internal standard as well as for relative quantification compared to the expression of housekeeping genes.

Also, another method for quantifying the initial concentration of a nucleic acid from the nucleic acid amplification data is disclosed in U.S. Patent Application Publication No. 2002-0031768. Herein, the concentration of the nucleic acid is quantified using a specific value of the derivative.

It is the object of the present invention to provide a method for quantifying the initial concentration of a nucleic acid from real-time nucleic acid amplification data without differentiation or integration.

This object is solved by the subject matter of the independent claims.

Preferred embodiments are defined by the dependent claims.

According to another aspect of the present invention, a method for quantifying the initial concentration of a nucleic acid includes: amplifying a nucleic acid; producing a function representing a correlation between fluorescence intensity which is proportional to the amount of the nucleic acid and amplification cycle number or amplification time of the nucleic acid; using the function to calculate the prior-to-amplification fluorescence intensity of the nucleic acid subtracted by a background fluorescence intensity of the nucleic acid; and calculating the initial concentration of the nucleic acid from the calculated prior-to-amplification fluorescence intensity subtracted by a background fluorescence intensity.

According to the present invention, the initial concentration of the nucleic acid can be quantified without differentiation or integration.

The above and other features and advantages of the present invention will become more apparent by describing in detail exemplary embodiments thereof with reference to the attached drawings in which:

FIG. 1 is a graph showing a mathematical model of a correlation between the fluorescence intensity of the amplified nucleic acid and amplification cycle number;

FIGS. 2A through 2F are graphs showing the real amplification data of a nucleic acid and the least-square fitting results of those data with the mathematical model of FIG. 1;

FIG. 3 is a graph showing the relation between amplification efficiency and amplification cycle number for various initial concentrations of a nucleic acid;

FIG. 4 is a graph showing a method of calculating the characteristic amplification cycle number at which amplification efficiency has the maximum value in FIG. 3;

FIG. 5 is a graph showing the relations of the characteristic amplification cycle number ($n_{1/2}$) at which the fluorescence intensity of the nucleic acid subtracted by the background fluorescence intensity of the nucleic acid has half of its maximum value in the mathematical model of FIG. 1 with the initial concentration of the nucleic acid, the characteristic amplification cycle number($n_{Emax}$) at which amplification efficiency has the maximum value in FIG. 3 with the initial concentration of the nucleic acid, and the conventional characteristic amplification cycle number $C_t$ where the $2^{nd}$ derivative of the fluorescence intensity has the maximum value with the initial concentration of the nucleic acid;

FIG. 6 is a graph showing the relation between $R_0$, the prior-to-amplification fluorescence intensity of the nucleic acid subtracted by a background fluorescence intensity of the nucleic acid in the mathematical model of FIG. 1 and the initial concentration of the nucleic acid;

FIGS. 7A through 7C are tables showing the method for calculating the initial concentration of the nucleic acid according to the present application and the result of the prior art;

FIGS. 8A through 8E are graphs showing variations of the relation between fluorescence intensity of a nucleic acid and amplification cycle number, and graphs showing variations of the relation between amplification efficiency and amplification cycle number for various initial concentrations of the nucleic acid;

FIG. 9 is a graph showing a relation between the characteristic amplification cycle number at which amplification efficiency has the maximum value and the initial concentration of a nucleic acid obtained from the test results of FIGS. 8A through 8E;

FIG. 10 is a graph showing variations of the relation between background-corrected amplification efficiency and amplification cycle number, adopting various values as the background fluorescence intensity of a nucleic acid;

FIGS. 11A through 11E are graphs showing variations of initial behaviours of fluorescence intensity of a nucleic acid for various initial concentrations;

FIGS. 12A and 12F are graphs showing variations of the background-corrected amplification efficiency profiles for various initial concentrations of a nucleic acid adopting various values as the background fluorescence intensity;

FIGS. 12G and 12H shows graphs of variations of the relation between the initial concentration of a nucleic acid and the characteristic amplification cycle number where the background-corrected amplification efficiency has the maximum or the minimum value, and graphs of variations of the relation between the initial concentration of a nucleic acid and %CV of the characteristic amplification cycle number where the background-corrected amplification efficiency has the maximum or the minimum value for various values of $R_b$ in FIGS. 12A and 12F;

FIG. 13 is a graph showing variations of the relations between fluorescence intensity and amplification cycle number for various methods of adopting the background fluorescence intensity;

FIGS. 14A and 14B show graphs showing variations of the relations between the background-corrected amplification efficiency and amplification cycle number for various methods of adopting the background fluorescence intensity in FIG. 13;

FIG. 15 is a graph showing the relations between the initial concentration of a nucleic acid and characteristic amplification cycle number where the background-corrected amplification efficiency has the maximum value with and without background fluorescence intensity correction;

FIG. 16 shows the effect of background fluorescence intensity correction on the accuracy of the quantification method of the initial concentration of a nucleic acid using the characteristic amplification cycle number where the background-corrected amplification efficiency has the maximum value, which is obtained using the relations of FIG. 15; and

FIG. 17 is a graph showing an example illustrating the effect of %CV of the characteristic amplification cycle number on the accuracy of quantification of the initial concentration of a nucleic acid.

A method for quantifying the initial concentration of a nucleic acid from a real-time nucleic acid amplification data, especially, a PCR data will now be described with reference to the accompanying drawings.

FIG. 1 is a graph showing a mathematical model of the relation between the fluorescence intensity of the amplified nucleic acid and amplification cycle number.

Nucleic acid amplification using enzyme is performed by [a] methods requiring a thermal cycle, such as PCR, RT-PCR, nested PCR and LCR, or isothermal nucleic acid amplification methods, such as SDA, NASBA, TMA and RCA. Here, the nucleic acid may be extracted from an organism or a virus.

The result of measuring the fluorescence intensity of the nucleic acid at each amplification cycle from a real-time PCR experiment can be modelled as the graph shown in FIG. 1. A sigmoidal model of FIG. 1 can be expressed by Equation 1 below.

**[Equation 1]**

$$R = R_b + \frac{R_{max}}{1 + e^{-\frac{n - n_{1/2}}{k}}}$$

where,

R: fluorescence intensity of a nucleic acid
$R_b$: background fluorescence intensity of a nucleic acid
$R_{max}$: the maximum fluorescence intensity of a nucleic acid
n: amplification cycle number
$n_{1/2}$: characteristic amplification cycle number at which the fluorescence intensity of the nucleic acid subtracted by the background fluorescence intensity of the nucleic acid has half of its maximum value
k: a parameter related to the rate of change of the fluorescence intensity during amplification reaction

The error between the mathematical model and the actual PCR data can be calculated by Equation 2 below.

$$\varepsilon^2 = \sum_n [R_n - \{R_b + \frac{R_{max}}{1+e^{-\frac{(n-n_{1/2})}{k}}}\}]^2$$

Using the least-square fitting method in order to compute the parameters $R_b$, $R_{max}$, $n_{1/2}$ and k in the mathematical model of FIG. 1, nonlinear-equation set of Equation 3 or Equations 4a through 4d is obtained and this can be solved using the Newton-Raphson method.

[Equation 3]

$$\frac{\partial\varepsilon^2}{\partial R_b} = 0,\ \frac{\partial\varepsilon^2}{\partial R_{max}},\ \frac{\partial\varepsilon^2}{\partial n_{1/2}} = 0,\ \frac{\partial\varepsilon^2}{\partial k} = 0$$

[Equation 4a]

$$\sum_n [R_n - \{R_b + \frac{R_{max}}{1+e^{-\frac{(n-n_{1/2})}{k}}}\}]^2 = 0$$

[Equation 4b]

$$\sum_n [R_n - \{R_b - \frac{R_{max}}{1+e^{-\frac{(n-n_{1/2})}{k}}}\} \times \frac{1}{1+e^{-\frac{n-n_{1/2}}{k}}}] = 0$$

[Equation 4c]

$$\sum_n [R_n - \{R_b + \frac{R_{max}}{1+e^{-\frac{(n-n_{1/2})}{k}}}\} \times \frac{e^{-\frac{n-n_{1/2}}{k}}}{\{1+e^{-\frac{n-n_{1/2}}{k}}\}^2}] = 0$$

[Equation 4d]

$$\sum_n [R_n - \{R_b - \frac{R_{max}}{1+e^{-\frac{(n-n_{1/2})}{k}}}\} \times \frac{(n-n_{1/2})e^{-\frac{n-n_{1/2}}{k}}}{\{1+e^{-\frac{n-n_{1/2}}{k}}\}^2}] = 0$$

When n=0 in Equation 1, the fluorescence intensity of the nucleic acid before the amplification reaction subtracted by the background fluorescence intensity $R_b$ can be defined by Equation 5.

[Equation 5]

$$R_0 = \frac{R_{max}}{1 + e^{n_{1/2}/k}}$$

FIGS. 2A through 2F are graphs showing the real amplification data of a HBV plasmid DNA and the least-square fitting results of those data with the mathematical model of FIG. 1.

FIG. 2A is the result when the initial concentration of the nucleic acid is $10^7$ copy/rxn (the number of copies in a reaction volume), FIG. 2B is the result when the initial concentration is $10^6$ copy/rxn, FIG. 2C is the result when the initial concentration is $10^5$ copy/rxn, FIG. 2D is the result when the initial concentration is $10^4$ copy/rxn, FIG. 2E is the result when the initial concentration is $10^5$ copy/rxn, and FIG. 2F is the result when the initial concentration is $2.5\times10^6$ copy/rxn. It can be seen that the cycle number at which the nucleic acid starts to be rapidly amplified decreases as the initial concentration of the nucleic acid increases.

Also, it can be seen that the graphs of the experimental results about the correlation between the fluorescence intensity of the nucleic acid and the amplification cycle number is almost similar to those of least-square fitting results using the mathematical model of Equation 1.

The amplification efficiency can be modelled as Equations 6a and 6b mathematically.

[Equation 6a]

$$R_n = (1 + E_n)R_{n-1}$$

where,

$R_n$: the fluorescence intensity at n-th amplification cycle
$R_{n-1}$: the fluorescence intensity at (n-1)-th amplification cycle
$E_n$: the amplification efficiency at n-th amplification cycle
Equation 6a can be rewritten as Equation 6b below.

[Equation 6b]

$$E_n = \frac{R_n - R_{n-1}}{R_{n-1}}$$

FIG. 3 is a graph showing the relation between the amplification efficiency and amplification cycle number for various initial concentrations of the nucleic acid. Referring to FIG. 3, it can be seen that the amplification efficiency is not constant during PCR cycles.

FIG. 4 is a graph showing a method of finding the characteristic amplification cycle number at which amplification efficiency has the maximum value in FIG. 3.

Referring to FIG. 4, assume that x-y coordinates at three points around the maximum or the minimum are known. The mathematical method of finding the x value which maximizes or minimizes y value using a parabolic curve fitting will now be described.

First, set Equation 7a below for three coordinates ($x_1$, $y_1$), ($x_2$, $y_2$) and ($x_3$, $y_3$).

[Equation 7a]

$$y1 = ax_1^2 + bx_1 + c,\ \ y2 = ax_2^2 + bx_2 + c,\ \ y3 = ax_3^2 + bx_3 + c$$

If Equation 7a is rewritten in a matrix form, the result is given by Equation 7b.

[Equation 7b]

$$\begin{pmatrix} x_1^2 & x_1 & 1 \\ x_2^2 & x_2 & 1 \\ x_3^2 & x_3 & 1 \end{pmatrix}\begin{pmatrix} a \\ b \\ c \end{pmatrix} = \begin{pmatrix} y_1 \\ y_2 \\ y_3 \end{pmatrix}$$

If one defines $\begin{vmatrix} x_1^2 & x_1 & 1 \\ x_2^2 & x_2 & 1 \\ x_3^2 & x_3 & 1 \end{vmatrix} = \det(A)$, constants a, b and c can be represented by Cramer's rule as follows.

[Equation 7c]

$$a = \begin{vmatrix} y_1 & x_1 & 1 \\ y_2 & x_2 & 1 \\ y_3 & x_3 & 1 \end{vmatrix} / \det(A)$$

$$b = \begin{vmatrix} x_1^2 & y_1 & 1 \\ x_2^2 & y_2 & 1 \\ x_3^2 & y_3 & 1 \end{vmatrix} / \det(A)$$

$$c = \begin{vmatrix} x_1^2 & x_1 & y_1 \\ x_2^2 & x_2 & y_2 \\ x_3^2 & x_3 & y_3 \end{vmatrix} / \det(A)$$

Then, $x_{max}$ can be calculated from Equation 7d below.

[Equation 7d]

$$x_{max} = -\frac{b}{2a} = \frac{y_1(x_2^2 - x_3^2) + y_2(x_3^2 - x_1^2) + y_3(x_1^2 - x_2^2)}{2[y_1(x_2 - x_3) + y_2(x_3 - x_1) + y_3(x_1 - x_2)]}$$

Hitherto, the method of implementing the relationships between the fluorescence intensity of the nucleic acid and the amplification cycle number using mathematical models and calculating the parameters of mathematical models have been described. A description will now be made about the relationships between a specific parameter of mathematical models and the initial concentration of the nucleic acid. Also, a description will be made about methods of quantification of the initial concentration of the nucleic acid based on the relationship.

FIG. 5 is a graph showing the relations of the characteristic amplification cycle number ($n_{1/2}$) at which the fluorescence intensity of the nucleic acid subtracted by the background fluorescence intensity of the nucleic acid has half of its maximum value in the mathematical model of FIG. 1 with the initial concentration of the nucleic acid, the characteristic amplification cycle number($n_{Emax}$) at whcich the amplification efficiency has the maximum value in FIG.

3 with the initial concentration of the nucleic acid, and the conventional characteristic amplification cycle number $C_t$ at which the 2nd derivative of the fluorescence intensity has the maximum value with the initial concentration of the nucleic acid.

Referring to FIG. 5, as the initial concentration (log[copy/rxn]) is higher, both characteristic amplification cycle numbers $n_{1/2}$ and $n_{Emax}$ decrease. Also, it can be seen that the slope of the conventional method of calculating the initial concentration of the nucleic acid using [a] the 2nd derivative [value] is almost the same to those using $n_{1/2}$ and $n_{Emax}$, which will be used in this application. Here, the standard calibration curves using $n_{Emax}$ is placed below those using $n_{1/2}$. Therefore, when $n_{Emax}$ is used, the initial concentration of the nucleic acid can be quantified within a smaller amplification cycle number than the case using $n_{1/2}$.

FIG. 6 is a graph showing the relation between $R_0$ in Equation 5 and the initial concentration of the nucleic acid. Referring to FIG. 6, it can be seen that log (initial concentration of nucleic acid) is linearly proportional to log($R_0$).

Three novel methods are described for obtaining the initial concentration of the nucleic acid using the proportional relationship of FIGS. 5 and 6. The first method is to use the characteristic amplification cycle number or time $n_{1/2}$ at which the fluorescence intensity of the nucleic acid subtracted by the background fluorescence intensity of the nucleic acid has half of its maximum value, and the second method is to use the characteristic amplification cycle number or the characteristic amplification time at which the amplification efficiency of the nucleic acid becomes maximum($n_{Emax}$) or minimum($n_{Emin}$). The third method is to use $R_0$, the prior-to-amplification fluorescence intensity subtracted by the background fluorescence intensity.

A description will now be made about methods for quantifying the initial concentration of the nucleic acid whose initial concentration is unknown, using the above novel methods.

First, a nucleic acid amplification reaction (for example, PCR, LCR, SDA, NASBA, TMA, RCA, etc.) is performed on predetermined standard nucleic acid samples, whose initial concentrations are known. Then, the parameters of the mathematical model shown in FIG. 1 or 3 are calculated. Then, the standard calibration curve like FIG. 5 or 6, is obtained using a parameter among $n_{1/2}$, $n_{Emax}$ and $R_0$.

Simultaneously, the same nucleic acid amplification reaction is performed on unknown samples and the same parameter($n_{1/2}$ or $n_{Emax}$ or $R_0$) as used in the standard calibration curve is calculated. Then, the initial concentration of the unknown nucleic acid samples [is] are found from the standard calibration curve shown in FIG. 5 or 6. Specifically, when $n_{Emax}$ is used, the amplification efficiency can be obtained and displayed in real time with much less numerical efforts. Moreover, the initial concentration of the nucleic acid can be quantified with a very simple calculation, thereby reducing the number of the amplification cycle number needed to quantify an unknown nucleic acid sample.

FIGS. 7A through 7C are tables showing an example of quantification results of the nucleic acid using the methods in the present application and those using $C_t$ from the 2nd derivative of the fluorescence intensity in the prior art.

FIG. 7A shows the calculated values of parameters used in quantification of the nucleic acid, whose initial concentrations are $10^4$, $10^5$, $10^6$ and $10^7$ copy/rxn so as to obtain the standard calibration curve, as shown in FIGS. 5 and 6. (6 repetitions) FIGS. 7B and 7C show the quantification results of [the] trial nucleic acid samples using the standard calibration curve obtained in FIG. 7A according to the present application, and those using the standard calibration curve in the prior art. Referring to FIGS. 7B and 7C, instead of Ct, $n_{1/2}$, $n_{Emax}$ or $R_o$ can be used as the characteristic factor for the quantitative analysis of the nucleic acid. In FIG. 7B, Error is the percentage of a value obtained by dividing an absolute value of [(the copy/rxn value calculated from the calibration curve)-(true copy/rxn value)] by (true copy/rxn value). For example, the first value in Error(Ct) is calculated as (5.4E+0.5 - 5.0E+05) / 5.0E+05, i.e., 8.08%. In FIG. 7C, Avg error at 5.0E05 represents an average value of errors in FIG. 7B from 6-time repetitions.

FIGS. 8A through 8E show graphs showing variations of the relation between fluorescence intensity of a nucleic acid and amplification cycle number, and graphs showing variations of the relation between amplification efficiency and amplification cycle number for initial concentrations $10^0$, $10^1$, $10^2$,$10^3$, $10^4$, $10^5$, $10^6$, $10^7$, and $10^8$ copy/rxn of the nucleic acid (9 repetitions).

Referring to FIGS. 8A through 8E, the background fluorescence signals of the nucleic acid are subtracted in the graphs showing the relations between the fluorescence intensity and the amplification cycle number. As can be seen from FIGS. 11A through 11E, the fluorescence intensity starts from a positive value, decreases and again increases at the initial stages. This results from the photobleaching effect, the well-known characteristics of a fluorescence dye. All the minimum fluorescence intensities of FIGS. 11A through 11E are zero because the background fluorescence intensities are subtracted. This causes the amplification efficiency profiles to have a pointed and sharp maximum as in FIGS. 8A through 8E. The maximum efficiencies in FIGS. 8A through 8E are larger than 1.0, which cannot be accepted as a concept of efficiency physically.

FIG. 9 is a graph which shows the relation between the characteristic amplification cycle number $n_{Emax}$ and the initial concentration of the nucleic acid obtained from the test result of FIGS. 8A through 8E.

Referring to FIG. 9, the graph is linear in range of initial concentration of the nucleic acid from $10^3$ to $10^7$ so that it can be used as a standard calibration curve for that range to calculate the initial concentration according to the present application. However, when the initial concentration of the nucleic acid is below $10^3$, the data have an irregular pattern.

In order to prevent the amplification efficiency profile from having a pointed and sharp maximum which is larger than 1.0, Equation 6b which represents the amplification efficiency can be rewritten as below.

**[Equation 8]**

$$E_n = \frac{(R_n + R_b) - (R_{n-1} + R_b)}{R_{n-1} + R_b} = \frac{R_n - R_{n-1}}{R_{n-1} + R_b}$$

where, $R_n$ is the fluorescence intensity in the n-th amplification cycle, $R_{n-1}$ is the fluorescence intensity in the (n-1)-th amplification cycle, $R_b$ is an arbitrary constant which represents the background fluorescence intensity, and $E_n$ is the amplification efficiency in the n-th amplification cycle, respectively.

FIG. 10 is a graph of the background-corrected amplification efficiency profiles according to four different definitions of the background fluorescence intensity $R_b$ in six repetitive tests when the initial concentration of the nucleic acid is $10^7$ copy/rxn. When $R_b$ is zero, that is, when the minimum value $R_{min}$ of the fluorescence intensity during amplification reaction is zero, the amplification efficiency profile has a pointed and sharp shape, as in FIGS. 8A through 8E. Although the amplification cycle numbers at which the maximum amplification efficiency occurs seem almost constant when $R_b$ is zero, it occurs at earlier cycle numbers and its value is not constant for 6 repetitions.

On the contrary, when $R_b$ is non-zero (that is, $R_1$, 0.01, $-(R_b)_{sigmoidal}$), the amplification cycle numbers at which the maximum amplification efficiency occurs seem almost constant and the value of the maximum amplification efficiency is also constant for 6 repetitions.

The constant acceptable as the constant $R_b$ and the improvement when $R_b$ is considered in the definition of the amplification efficiency will now be described with reference to FIGS. 15 through 17.

First, $-(R_b)_{sigmoidal}$, which results from the least-square fitting of the fluorescence intensity data at all amplification cycles to sigmoidal model in Equation 1, can be used as $R_b$ in Equation 8.

Second, when the fluorescence intensity starts from non-zero, decreases to zero and again increases (refer to FIGS. 11A through 11E) at the initial stage of amplification reaction, the value corresponding to the initial fluorescence intensity can be used as the value of $R_b$. For example, referring to FIGS. 11A through 11E, although the initial fluorescence intensity is slightly different for different initial concentrations of the nucleic acid, it is 0.01 on average. Therefore, the value of 0.01 can be used as the value of $R_b$. The initial fluorescence intensity will not change significantly if the amplification conditions such as reagents, fluorescence dyes, and optical exposure time do not change. Therefore, once the value corresponding to the initial fluorescence intensity is determined experimentally, it can be used as $R_b$ from that time on. On the contrary, if the amplification conditions such as reagents, fluorescence dyes, and optical exposure time change, the value corresponding to the initial fluorescence intensity must be determined again experimentally.

Third, the fluorescence intensity at the first amplification cycle, $R_1$ can be used as the value of $R_b$. Even though the amplification conditions such as reagents, fluorescence dyes, and optical exposure time change, this method has the advantage that users need no additional efforts for determining $R_b$ because the fluorescence intensity at the first amplification cycle is always measured at every test. Although this method has this advantage over the second method, the quantification error may increase when the fluorescence intensity at the first amplification cycle is very close to zero.

Fourth, the value that makes the maximum amplification efficiency be "1" (unity) in the amplification efficiency function of Equation 8 can be used as the value of $R_b$. In order to obtain $R_b$ such that the maximum amplification efficiency is "1", the value of $R_b$ has to be found by iterative methods such as successive substitution at every amplification experiment. Although this method is ideal in principle, the iterative calculation may be complicated or time-consuming.

As described above, the background fluorescence intensity $R_b$ can be set to one of the following values: 1) $-(R_b)_{sigmoidal}$ obtained through the least-square fitting of the fluorescence intensity data at all amplification cycles to the sigmoidal model in Equation 1; 2) the value (about 0.01 for FIGS. 11A through 11E) corresponding to the initial fluorescence intensity; 3) [the strength $R_1$ of] the fluorescence intensity at the first amplification cycle, $R_1$; and 4) the value that makes the maximum amplification efficiency be "1".

Here, the use of $-(R_b)_{sigmoidal}$ of the background fluorescence intensity may be inconvenient because it must be calculated through the nonlinear curve fitting of sigmoidal model. However, other methods in the present invention (the initial fluorescence intensity, $R_1$, and the value that makes the maximum amplification efficiency be "1") need not use the curve fitting.

Even a larger positive or negative value can be used as the value of $R_b$ for calculating the background-corrected amplification efficiency. This will be described with reference to FIGS. 12A through 12H.

FIGS. 12A through 12C and 12D through 12F are graphs illustrating variations of the background-corrected amplification efficiency for various values of the background fluorescence intensity using the same experimental data as in FIGS. 8A through 8E.

The cases where $R_b$ are positive values of 0.01, 0.1, 1, 10, 100, and 1000 are shown in FIGS. 12A through 12C. Since the respective cases have well-distinguishable positive peaks in background-corrected amplification efficiency with almost an equal interval in amplification cycle number according to the initial concentration of the nucleic acid, they can be used to quantify the initial concentration of the nucleic acid according to the present invention. However, when the value of $R_b$ is larger than 0.1, the maximum value of the background-corrected amplification efficiency becomes very smaller than 1, which has no physical meaning in terms of the amplification efficiency. However, as can be seen from FIGS. 12A through 12C, the amplification cycles $n_{Emax}$ where the respective background-corrected amplification efficiency profiles have the maximum values are located at almost an equal interval. Thus, all of them can be used in qualifying the initial concentration of the nucleic acid. However, it is preferable to use the case where the background-corrected amplification efficiency is in range from 0 to 1, that is, the case where the value of $R_b$ is 0.01 (the value corresponding to the initial fluorescence intensity of the nucleic acid).

The cases where $R_b$ are negative values of -0.01, -0.1, -1, -10, -100, and -1000 are shown in FIGS. 12D through 12F. When the value of $R_b$ is -0.01 and -0.1, the divisor of the background-corrected amplification efficiency function (Equation 8) approaches to zero very closely, so that the background-corrected amplification efficiency profiles have a pointed and sharp peaks and do not represent the regular-interval pattern when the absolute value of $R_b$ is large, the background-corrected amplification efficiency profiles have well-distinguishable negative peaks with almost an equal interval in amplification cycle number according to the initial concentration of the nucleic acid. Specifically, when the value of $R_b$ is less than -1, the value of the background-corrected amplification efficiency is negative. Since the amplification efficiency has the physical meaning only in the range from 0 to 1, although the initial concentration of the nucleic acid can be quantified using the values given in FIGS. 12D through 12F, there is no physical meaning in terms of the amplification efficiency. However, as can be seen from FIG. 12B, when the value of $R_b$ is less than -1 (e.g., -1, -10, -100, -1000), the amplification cycles $n_{Emin}$ where the respective background-corrected amplification efficiency profiles have the minimum values are located at almost an equal interval. Thus, all of them can be used in qualifying the initial concentration of the nucleic acid.

FIGS. 12G and 12H show graphs of variations of the relation between the initial concentration of the nucleic acid and the characteristic amplification cycle number $n_{Emax}$ or $n_{Emin}$ where the background-corrected amplification efficiency has the maximum or the minimum value, and graphs of variations of the relation between the initial concentration of a nucleic acid and %CV of $n_{Emax}$ or $n_{Emin}$ for various values of $R_b$ in FIGS. 12A through 12C and 12D through 12F.

Referring to FIGS. 12G and 12H, when the value of $R_b$ increases to a positive value (FIGS. 12A through 12C) or decreases to a negative value (FIGS. 12D through 12F), the graphs of the relation between the initial concentration of the nucleic acid and $n_{Emax}$ or $n_{Emin}$ converge to a curve, respectively. Except the case where the value of $R_b$ is 0 and -0.01, %CV of $n_{Emax}$ is within 5% when the initial concentration of the nucleic acid is in range from $10^3$ to $10^8$ copy/rxn. A relation between the quantification error in the initial concentration of the nucleic acid and %CV of $n_{Emax}$ or $n_{Emin}$ will be described with reference to FIG. 17.

When the value of $R_b$ is larger than some value, the value of %CV does not almost change. Thus, the values except $R_b$=0 in FIG. 10, that is, $R_1$, 0.01, and $-(R_b)_{sigmoidal}$ may be preferably used as the value of $R_b$ so as to improve %CV of $n_{Emax}$ while the amplification efficiency is in range from 0 to 1.

FIG. 13 is a graph showing variations of the relations between fluorescence intensity and amplification cycle number for various methods of adopting the background fluorescence intensity.

FIGS. 14A and 14B show graphs showing variations of the relations between the background-corrected amplification efficiency and amplification cycle number for various methods of adopting the background fluorescence intensity in FIG. 13.

Referring to FIGS. 13 and 14A, when the value of $R_b$ is zero, which is the minimum value of the fluorescence intensity profiles (that is, $R_b=R_{bmin}=0$), so that the divisor $(R_{n-1}+R_b)$ of Equation 8 approaches to zero. Thus, the amplification efficiency $E_n$ increases too large and $E_{max}$ peak becomes sharp and pointed. Also, the amplification efficiency may be easily influenced by noise. When the value of $R_b$ is non-zero, the amplification efficiency profiles have well-distinguishable peaks with almost an equal interval in amplification cycle number according to the initial concentration of the nucleic acid. However, in FIG. 14B, if $R_b=R_1$, the maximum values of the background-corrected amplification efficiency are larger than 1 in the initial stage of the amplification cycle (cycle number$\leq$ 5) and the shape of the peaks are very sharp and pointed. In the conventional method of amplifying the nucleic acid, this rapid amplification in this early stage generally does not occur and the amplification efficiency of larger than 1 has no physical meaning. Therefore, these peaks have to be ignored and the second maximum peaks could be used for quantification of the initial concentration of the nucleic acid.

FIG. 15 is a graph showing the standard calibration curves between the initial concentration of the nucleic acid and the characteristic amplification cycle number $n_{Emax}$ with ($R_b$=0.01) and without ($R_b$=0) the background fluorescence intensity correction.

Referring to FIG. 15, both standard calibration curves (with and without the background fluorescence correction) are linear in the initial concentration of the nucleic acid ranging from $10^3$ to $10^8$. [The latter graph is disposed above the former graph.] It is seen that he standard calibration curve with background fluorescence correction is located above that without background fluorescence correction.

FIG. 16 shows the effect of background fluorescence intensity correction on the accuracy of the quantification method of the initial concentration of the nucleic acid using the characteristic amplification cycle number $n_{Emax}$, which is obtained using the standard calibration curve of FIG. 15.

As can be seen from FIG. 16, using the background fluorescence correction ($R_b$=0.01), %CV (=St.Dev(standard deviation)/Avg(average) of $n_{Emax}$ is reduced remarkably.

FIG. 17 is a graph showing an example illustrating the effect of %CV of the characteristic amplification cycle number on the accuracy of quantification of the initial concentration of a nucleic acid.

Referring to FIG. 17, when %CV of the characteristic amplification cycles changes from 0.5 to 5.0, the error (in copy) greatly changes from 6.85% to 48.5%. That is, even a slight increase in the error of the characteristic amplification cycle may cause a large error in the quantification results of the initial concentration of the nucleic acid. For these reasons, a very accurate quantifying method is indispensable. As shown in FIG. 16, using the background fluorescence correction ($R_b$=0.01), the %CV of $n_{Emax}$ is greatly reduced so that the quantification accuracy is greatly improved compared with the case without using the background fluorescence correction ($R_b$=0).

According to the present application, three novel methods for finding the initial concentration of the nucleic acid from the real-time nucleic acid amplification (PCR, LCR, SDA, NASBA, TMA, RCA, etc.) data are described using the characteristic amplification cycle number or time at which the fluorescence intensity of the nucleic acid subtracted by the background fluorescence intensity of the nucleic acid has half of its maximum value, the characteristic amplification cycle number or time at which the amplification efficiency has the maximum or the minimum value, and the prior-to-amplification fluorescence intensity subtracted by the background fluorescence intensity, based on the mathematical model with respect to the correlation between the amplification amount of the nucleic acid and the amplification cycle, without differentiation or integration.

Specifically, when the characteristic amplification cycle at which the amplification efficiency has the maximum or the minimum value is used, the amplification efficiency can be obtained and displayed in real time with much less numerical efforts from the real-time nucleic acid amplification data. Therefore, the initial concentration of the nucleic acid can be found more rapidly than other methods.

The invention can also be embodied as computer readable codes on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, optical data storage devices, and carrier waves (such as data transmission through the Internet). The computer-readable recording medium can also be distributed over network coupled computer systems so that the computer readable code is stored and executed in a distribution fashion.

## Claims

1. A method for quantifying an initial concentration of a nucleic acid from real-time nucleic acid amplification data comprising:

    amplifying a nucleic acid;
    producing a function representing a correlation between a fluorescence intensity which increases or decreases in proportion to an amount of the nucleic acid and an amplification cycle number or an amplification time of the nucleic acid;
    using the function to calculate a prior-to-amplification fluorescence intensity at the 0-th amplification cycle subtracted by a background fluorescence intensity;

    wherein the initial concentration of the nucleic acid is calculated from the prior-to-amplification fluorescence intensity at the 0-th amplification cycle by using a standard calibration curve, which represents a relationship between the initial concentration of the nucleic acid and the prior-to-amplification fluorescence intensity subtracted by the background fluorescence intensity.

2. The method of claim 1, wherein the nucleic acid is amplified using enzyme.

3. The method of claim 1, wherein the nucleic acid is amplified using a nucleic acid amplification method that requires a thermal cycling.

4. The method of claim 1, wherein the nucleic acid is amplified using an isothermal nucleic acid amplification method.

5. The method of one of claims 1 to 4, wherein the function representing the correlation is produced using a sigmoidal model.

6. The method of claim 5. wherein the prior-to-amplification fluorescence intensity subtracted by the background fluorescence intensity is calculated using a nonlinear least-square fitting method with respect to the function that uses the sigmoidal model.

**Patentansprüche**

1. Verfahren zum Quantifizieren einer Anfangskonzentration einer Nukleinsäure aus Echtzeit-Nukleinsäure-Amplifikationsdaten, umfassend:

   Amplifizieren einer Nukleinsäure;
   Erstellen einer Funktion, die eine Korrelation zwischen einer Fluoreszenzintensität, welche proportional zu einer Menge der Nukleinsäure zunimmt oder abnimmt, und
   einer Amplifikationszyklusnummer oder einer Amplifikationszeit der Nukleinsäure beschreibt;
   Verwenden der Funktion zum Berechnen einer Vor-Amplifikations-Fluoreszenzintensität beim 0. Amplifikationszyklus minus einer Hintergrundfluoreszenzintensität;
   wobei die Anfangskonzentration der Nukleinsäure durch Verwenden einer Standard-Eichkurve, welche eine Beziehung zwischen der Anfangskonzentration der Nukleinsäure und der Vor-Amplifikations-Fluoreszenzintensität minus der Hintergrundfluoreszenzintensität darstellt, aus der Vor-Amplifikations-Fluoreszenzintensität beim 0. Amplifikationszyklus berechnet wird.

2. Verfahren nach Anspruch 1, wobei die Nukleinsäure durch ein Enzym amplifiziert wird.

3. Verfahren nach Anspruch 1, wobei die Nukleinsäure unter Verwendung eines Nukleinsäure-Amplifikationsverfahrens amplifiziert wird, das Temperaturzyklen erfordert.

4. Verfahren nach Anspruch 1, wobei die Nukleinsäure unter Verwendung eines isothermischen Nukleinsäure-Amplifikationsverfahrens amplifiziert wird.

5. Verfahren nach einem der Ansprüche 1 bis 4, wobei die Funktion, welche die Korrelation beschreibt, unter Verwendung eines sigmoidalen Modells erstellt wird.

6. Verfahren nach Anspruch 5, wobei die Vor-Amplifikations-Fluoreszenzintensität minus der Hintergrundfluoreszenzintensität berechnet wird unter Verwendung einer nicht-linearen Methode der kleinsten Fehlerquadrate in Bezug auf die Funktion, die das sigmoidale Modell verwendet.

**Revendications**

1. Procédé de quantification d'une concentration initiale d'un acide nucléique à partir de données d'amplification d'acide nucléique en temps réel, comprenant :

   amplifier un acide nucléique,
   produire une fonction représentant une corrélation entre une intensité de fluorescence qui augmente ou diminue en fonction d'une quantité de l'acide nucléique et d'un nombre de cycles d'amplification ou d'une durée d'amplification de l'acide nucléique,
   utiliser la fonction pour calculer une intensité de fluorescence avant amplification pendant le cycle 0 d'amplification, soustraite d'une intensité de fluorescence d'arrière plan,
   la concentration initiale de l'acide nucléique étant calculée en utilisant l'intensité de fluorescence avant amplification pendant le cycle 0 d'amplification en utilisant une courbe d'étalonnage standard qui représente une relation entre la concentration initiale de l'acide nucléique et l'intensité de fluorescence avant amplification soustraite de l'intensité de fluorescence de l'arrière plan.

2. Procédé selon la revendication 1, où l'acide nucléique est amplifié au moyen d'une enzyme.

**3.** Procédé selon la revendication 1, où l'acide nucléique est amplifié en utilisant un procédé d'amplification d'acide nucléique qui nécessite un cycle thermique.

**4.** Procédé selon la revendication 1, où l'acide nucléique est amplifié en utilisant un procédé d'amplification d'acide nucléique isothermique.

**5.** Procédé selon l'une des revendications 1 à 4, où la fonction représentant la corrélation est produite en utilisant un modèle sigmo ïdal.

**6.** Procédé selon la revendication 5, où l'intensité de la fluorescence avant amplification, soustraite de l'intensité de la fluorescence de l'arrière plan, est calculée en utilisant un procédé d'ajustement non linéaire suivant les moindres carrés par rapport à la fonction qui utilise le modèle sigmo ïdal.

FIG. 1

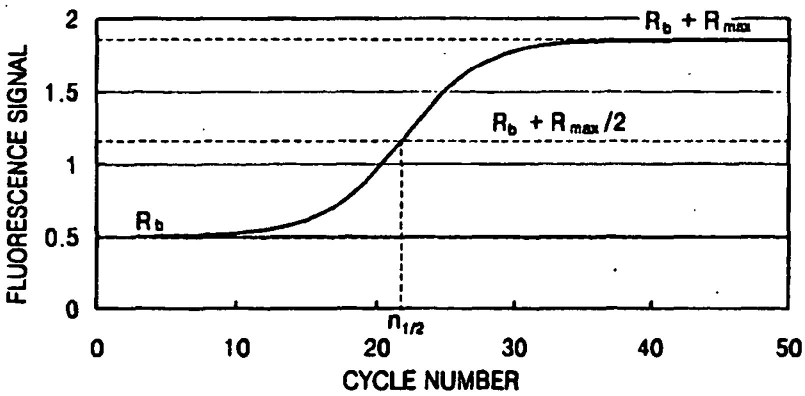

$R_b + R_{max}$
$R_b + R_{max}/2$
$R_b$
$n_{1/2}$
FLUORESCENCE SIGNAL
2
1.5
1
0.5
0
0 10 20 30 40 50
CYCLE NUMBER


FIG. 2A

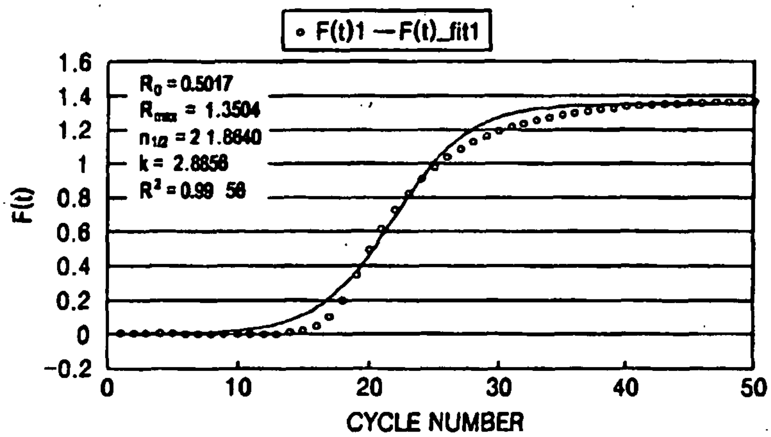

F(t)1 —F(t)_fit1
$R_0 = 0.5017$
$R_{max} = 1.3504$
$n_{1/2} = 2 1.8840$
k = 2.8858
$R^2 = 0.99 58$
F(t)
1.6
1.4
1.2
1
0.8
0.6
0.4
0.2
0
-0.2
0 10 20 30 40 50
CYCLE NUMBER

FIG. 2B

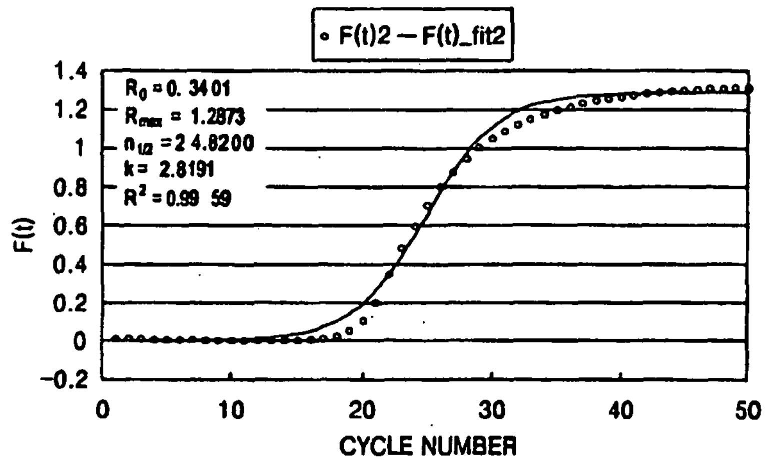
F(t)2 — F(t)_fit2
$R_0$ = 0.3401
$R_{max}$ = 1.2873
$n_{1/2}$ = 24.8200
k = 2.8191
$R^2$ = 0.9959
1.4
1.2
1
0.8
0.6
0.4
0.2
0
-0.2
F(t)
0
10
20
30
40
50
CYCLE NUMBER

FIG. 2C

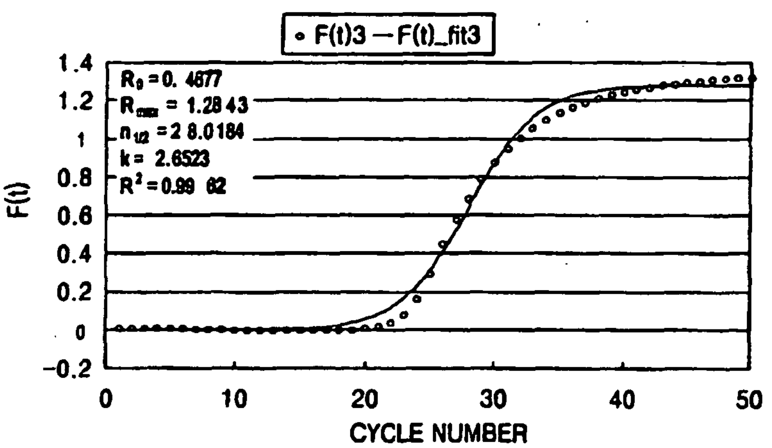
F(t)3 — F(t)_fit3
$R_0$ = 0.4877
$R_{max}$ = 1.2843
$n_{1/2}$ = 28.0184
k = 2.6523
$R^2$ = 0.9962
1.4
1.2
1
0.8
0.6
0.4
0.2
0
-0.2
F(t)
0
10
20
30
40
50
CYCLE NUMBER

FIG. 2D

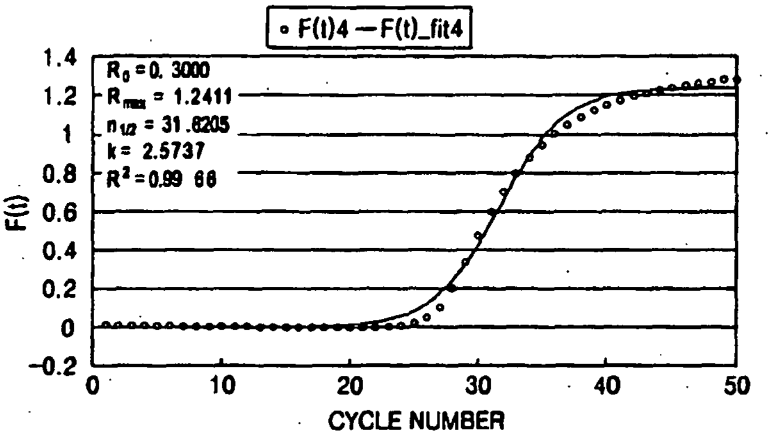

∘ F(t)4 —F(t)_fit4
$R_0$ = 0. 3000
$R_{max}$ = 1.2411
$n_{1/2}$ = 31 .6205
k= 2.5737
$R^2$ = 0.99 66
1.4
1.2
1
0.8
0.6
0.4
0.2
0
-0.2
F(t)
0
10
20
30
40
50
CYCLE NUMBER


FIG. 2E

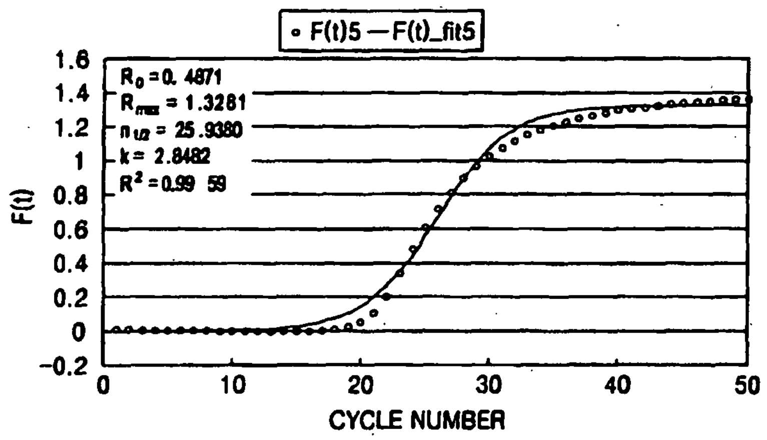

∘ F(t)5 —F(t)_fit5
$R_0$ = 0. 4871
$R_{max}$ = 1.3281
$n_{1/2}$ = 25 .9380
k= 2.8482
$R^2$ = 0.99 59
1.6
1.4
1.2
1
0.8
0.6
0.4
0.2
0
-0.2
F(t)
0
10
20
30
40
50
CYCLE NUMBER

FIG. 2F

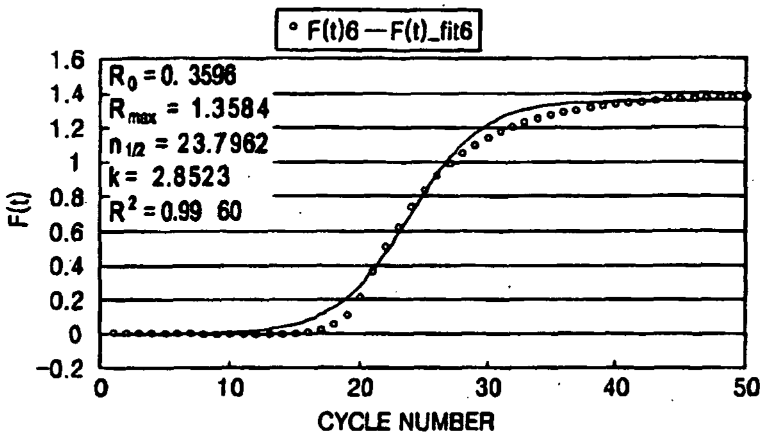

F(t)6 — F(t)_fit6
$R_0$ = 0. 3596
$R_{max}$ = 1.3584
$n_{1/2}$ = 23.7962
k = 2.8523
$R^2$ = 0.99 60
F(t)
CYCLE NUMBER

FIG. 3

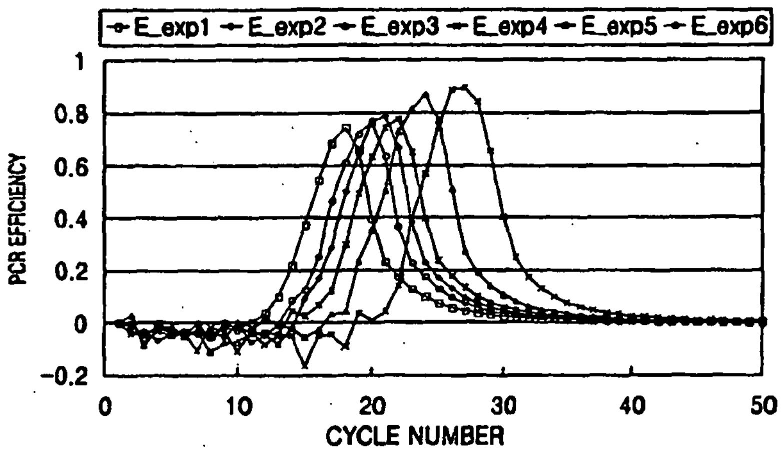
E_exp1
E_exp2
E_exp3
E_exp4
E_exp5
E_exp6
PCR EFFICIENCY
1
0.8
0.6
0.4
0.2
0
-0.2
0
10
20
30
40
50
CYCLE NUMBER

FIG. 4

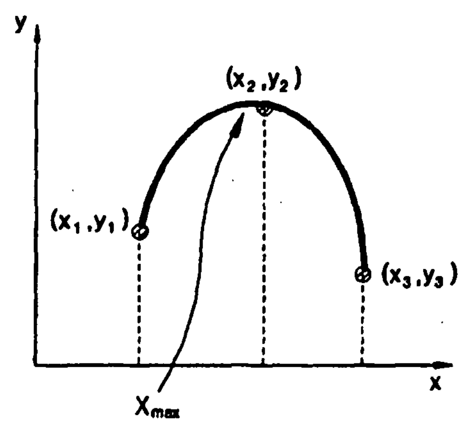
y
(x2,y2)
(x1,y1)
(x3,y3)
x
Xmax

FIG. 5

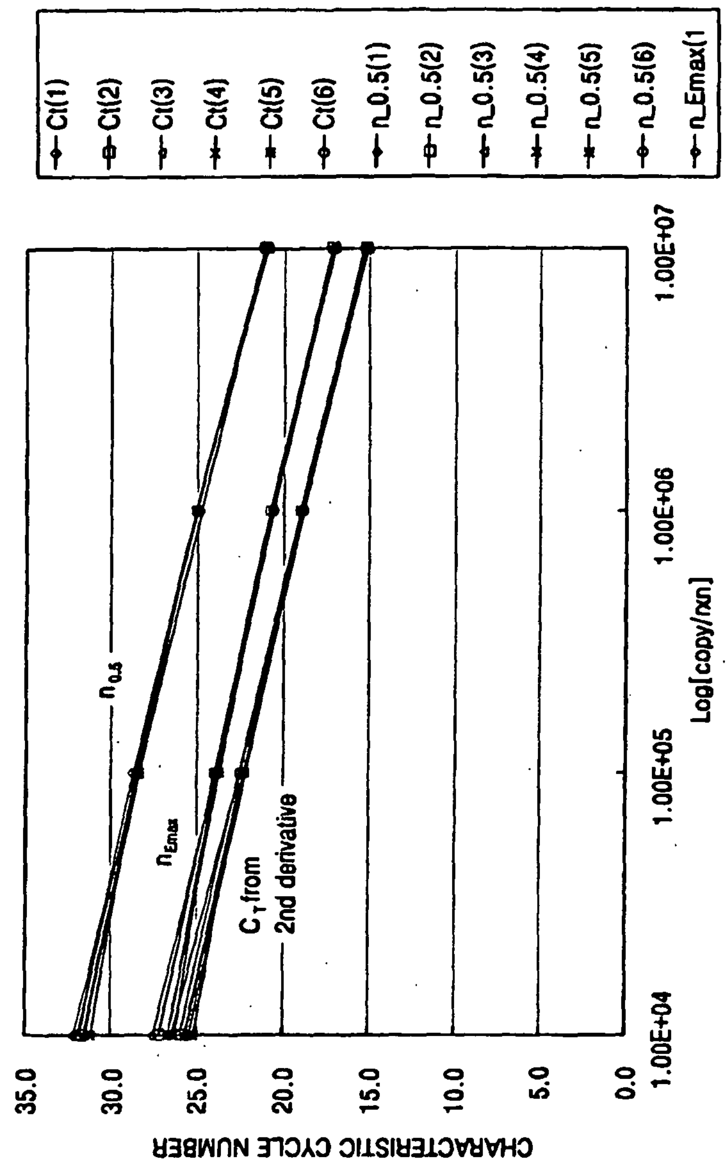
CHARACTERISTIC CYCLE NUMBER
35.0
30.0
25.0
20.0
15.0
10.0
5.0
0.0
1.00E+04
1.00E+05
1.00E+06
1.00E+07
Log[copy/rxn]
$n_{0.5}$
$n_{Emax}$
$C_T$ from
2nd derivative
Ct(1)
Ct(2)
Ct(3)
Ct(4)
Ct(5)
Ct(6)
n_0.5(1)
n_0.5(2)
n_0.5(3)
n_0.5(4)
n_0.5(5)
n_0.5(6)
n_Emax(1

FIG. 6

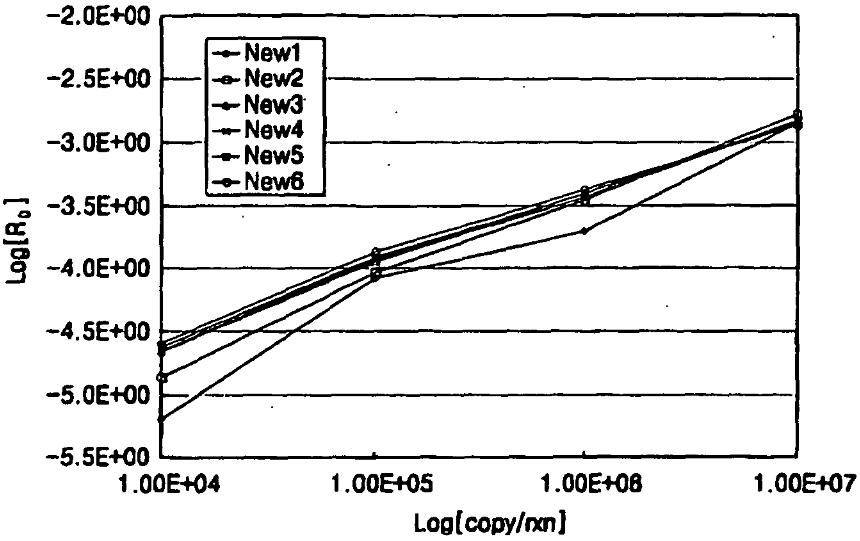
-2.0E+00
-2.5E+00
-3.0E+00
-3.5E+00
-4.0E+00
-4.5E+00
-5.0E+00
-5.5E+00
Log[R0]
New1
New2
New3
New4
New5
New6
1.00E+04
1.00E+05
1.00E+06
1.00E+07
Log[copy/rxn]

FIG. 7A

| Run Number | copy/rxn | Ct | n_0.5 | $Log_{10}$[R0] | n_Emax | Calibration curve (Ct) | Calibration curve (n_0.5) | Calibration curve ($Log_{10}$[R0]) | Calibration curve (n_Emax) |
|---|---|---|---|---|---|---|---|---|---|
| 1 | 1.00E+04 | 26.470 | 32.213 | -5.193 | 27.533 | Ct = -3.663 $Log_{10}$[copy] + 40.989 ($R^2$=0.999) | n_0.5 = -3.681 $Log_{10}$[copy] + 46.857 ($R^2$=1.000) | $Log_{10}$[R0] = 0.740 $Log_{10}$[copy] - 8.027 ($R^2$=0.951) | n_Emax = -3.410 $Log_{10}$[copy] + 41.064 ($R^2$=0.999) |
| | 1.00E+05 | 22.470 | 28.347 | -4.077 | 23.849 | | | | |
| | 1.00E+06 | 19.020 | 24.755 | -3.704 | 20.618 | | | | |
| | 1.00E+07 | 15.410 | 21.142 | -2.850 | 17.245 | | | | |
| 2 | 1.00E+04 | 25.960 | 31.843 | -4.861 | 27.153 | Ct = -3.554 $Log_{10}$[copy] + 40.247 ($R^2$=0.999) | n_0.5 = -3.556 $Log_{10}$[copy] + 48.214 ($R^2$=0.998) | $Log_{10}$[R0] = 0.684 $Log_{10}$[copy] - 7.542 ($R^2$=0.993) | n_Emax = -3.302 $Log_{10}$[copy] + 40.437 ($R^2$=0.999) |
| | 1.00E+05 | 22.510 | 28.559 | -4.033 | 23.959 | | | | |
| | 1.00E+06 | 19.070 | 25.064 | -3.456 | 20.776 | | | | |
| | 1.00E+07 | 15.260 | 21.154 | -2.774 | 17.206 | | | | |
| 3 | 1.00E+04 | 25.610 | 31.653 | -4.657 | 26.656 | Ct = -3.477 $Log_{10}$[copy] + 39.621 ($R^2$=0.997) | n_0.5 = -3.534 $Log_{10}$[copy] + 45.975 ($R^2$=0.996) | $Log_{10}$[R0] = 0.598 $Log_{10}$[copy] - 7.002 ($R^2$=0.994) | n_Emax = -3.198 $Log_{10}$[copy] + 39.644 ($R^2$=0.995) |
| | 1.00E+05 | 22.260 | 28.429 | -3.947 | 23.812 | | | | |
| | 1.00E+06 | 19.020 | 25.095 | -3.404 | 20.718 | | | | |
| | 1.00E+07 | 15.100 | 20.986 | -2.845 | 17.026 | | | | |
| 4 | 1.00E+04 | 25.170 | 31.175 | -4.847 | 26.274 | Ct = -3.287 $Log_{10}$[copy] + 38.568 ($R^2$=0.994) | n_0.5 = -3.378 $Log_{10}$[copy] + 45.058 ($R^2$=0.987) | $Log_{10}$[R0] = 0.595 $Log_{10}$[copy] - 6.981 ($R^2$=0.993) | n_Emax = -3.027 $Log_{10}$[copy] + 38.695 ($R^2$=0.989) |
| | 1.00E+05 | 22.430 | 28.643 | -3.929 | 23.941 | | | | |
| | 1.00E+06 | 18.990 | 24.998 | -3.436 | 20.724 | | | | |
| | 1.00E+07 | 15.360 | 21.135 | -2.829 | 17.258 | | | | |
| 5 | 1.00E+04 | 25.410 | 31.500 | -4.620 | 26.669 | Ct = -3.358 $Log_{10}$[copy] + 38.949 ($R^2$=0.998) | n_0.5 = -3.458 $Log_{10}$[copy] + 45.512 ($R^2$=0.995) | $Log_{10}$[R0] = 0.586 $Log_{10}$[copy] - 6.915 ($R^2$=0.993) | n_Emax = -3.127 $Log_{10}$[copy] + 39.270 ($R^2$=0.997) |
| | 1.00E+05 | 22.230 | 28.368 | -3.913 | 23.660 | | | | |
| | 1.00E+06 | 18.880 | 25.071 | -3.410 | 20.727 | | | | |
| | 1.00E+07 | 15.300 | 21.078 | -2.838 | 17.222 | | | | |
| 6 | 1.00E+04 | 25.650 | 31.945 | -4.600 | 26.595 | Ct = -3.470 $Log_{10}$[copy] + 39.595 ($R^2$=0.999) | n_0.5 = -3.651 $Log_{10}$[copy] + 46.784 ($R^2$=0.995) | $Log_{10}$[R0] = 0.570 $Log_{10}$[copy] - 6.813 ($R^2$=0.986) | n_Emax = -3.157 $Log_{10}$[copy] + 39.390 ($R^2$=0.998) |
| | 1.00E+05 | 22.270 | 28.765 | -3.871 | 23.756 | | | | |
| | 1.00E+06 | 18.920 | 25.106 | -3.373 | 20.643 | | | | |
| | 1.00E+07 | 15.200 | 20.994 | -2.866 | 17.109 | | | | |

FIG. 7B

| Run number | copy/ml | Ct | n_0.5 | Log(R0) | n_Emax | copy/ml(Ct) | copy/ml(n_0.5) | copy/ml(Log(R0)) | copy/ml(n_Emax) | Error(Ct) | Error(n_0.5) | Error(Log(R0)) | Error(n_Emax) |
|---|---|---|---|---|---|---|---|---|---|---|---|---|---|
| 1 | 5.00E+05 | 19.890 | 25.743 | -3.749 | 21.541 | 5.40E+05 | 5.45E+05 | 6.01E+05 | 5.32E+05 | 6.08% | 9.08% | 20.23% | 6.37% |
| | 2.50E+06 | 17.150 | 22.914 | -3.349 | 18.849 | 3.22E+06 | 3.20E+06 | 2.08E+06 | 3.28E+06 | 28.86% | 28.03% | 16.41% | 31.09% |
| 2 | 5.00E+05 | 19.880 | 25.692 | -3.780 | 21.623 | 5.01E+05 | 5.80E+05 | 3.17E+05 | 4.88E+05 | 0.19% | 17.98% | 38.54% | 0.47% |
| | 2.50E+06 | 17.030 | 23.177 | -3.061 | 18.925 | 3.41E+06 | 3.01E+06 | 3.57E+06 | 3.27E+06 | 38.36% | 20.23% | 42.72% | 30.63% |
| 3 | 5.00E+05 | 19.800 | 25.801 | -3.848 | 21.622 | 4.70E+05 | 5.12E+05 | 4.07E+05 | 4.31E+05 | 6.06% | 2.43% | 18.67% | 13.78% |
| | 2.50E+06 | 17.060 | 23.172 | -3.080 | 18.897 | 3.08E+06 | 2.84E+06 | 3.48E+06 | 3.07E+06 | 23.23% | 13.60% | 39.64% | 22.72% |
| 4 | 5.00E+05 | 19.840 | 25.629 | -3.645 | 21.447 | 4.88E+05 | 5.68E+05 | 4.07E+05 | 5.00E+05 | 0.46% | 13.59% | 18.59% | 0.01% |
| | 2.50E+06 | 17.020 | 23.177 | -3.058 | 18.885 | 3.56E+06 | 3.02E+06 | 3.95E+06 | 3.51E+06 | 43.54% | 20.84% | 57.83% | 40.62% |
| 5 | 5.00E+05 | 19.860 | 25.703 | -3.768 | 21.610 | 4.52E+05 | 5.39E+05 | 2.39E+05 | 4.44E+05 | 9.66% | 7.83% | 52.26% | 11.30% |
| | 2.50E+06 | 17.010 | 23.153 | -3.067 | 18.878 | 3.41E+06 | 2.85E+06 | 3.74E+06 | 3.32E+06 | 36.59% | 17.85% | 49.81% | 32.82% |
| 6 | 5.00E+05 | 19.770 | 25.492 | -3.681 | 21.438 | 5.17E+05 | 6.78E+05 | 3.11E+05 | 4.66E+05 | 3.34% | 35.67% | 37.73% | 2.77% |
| | 2.50E+06 | 16.970 | 23.356 | -3.002 | 18.820 | 3.31E+06 | 2.61E+06 | 4.84E+06 | 3.28E+06 | 32.51% | 4.41% | 93.41% | 31.04% |

FIG. 7C

| | Error(Ct) | Error(n_0.5) | Error(Log[R0]) | Error(n_Emax) |
|---|---|---|---|---|
| Avg error at 5.0E5 | 4.63% | 14.43% | 30.67% | 5.78% |
| Avg error at 2.5E6 | 33.51% | 17.53% | 49.93% | 31.47% |
| StDev error at 5.0E5 | 3.95% | 11.67% | 13.78% | 5.75% |
| StDev error at 2.5E6 | 7.02% | 7.96% | 25.44% | 5.68% |

FIG. 8A

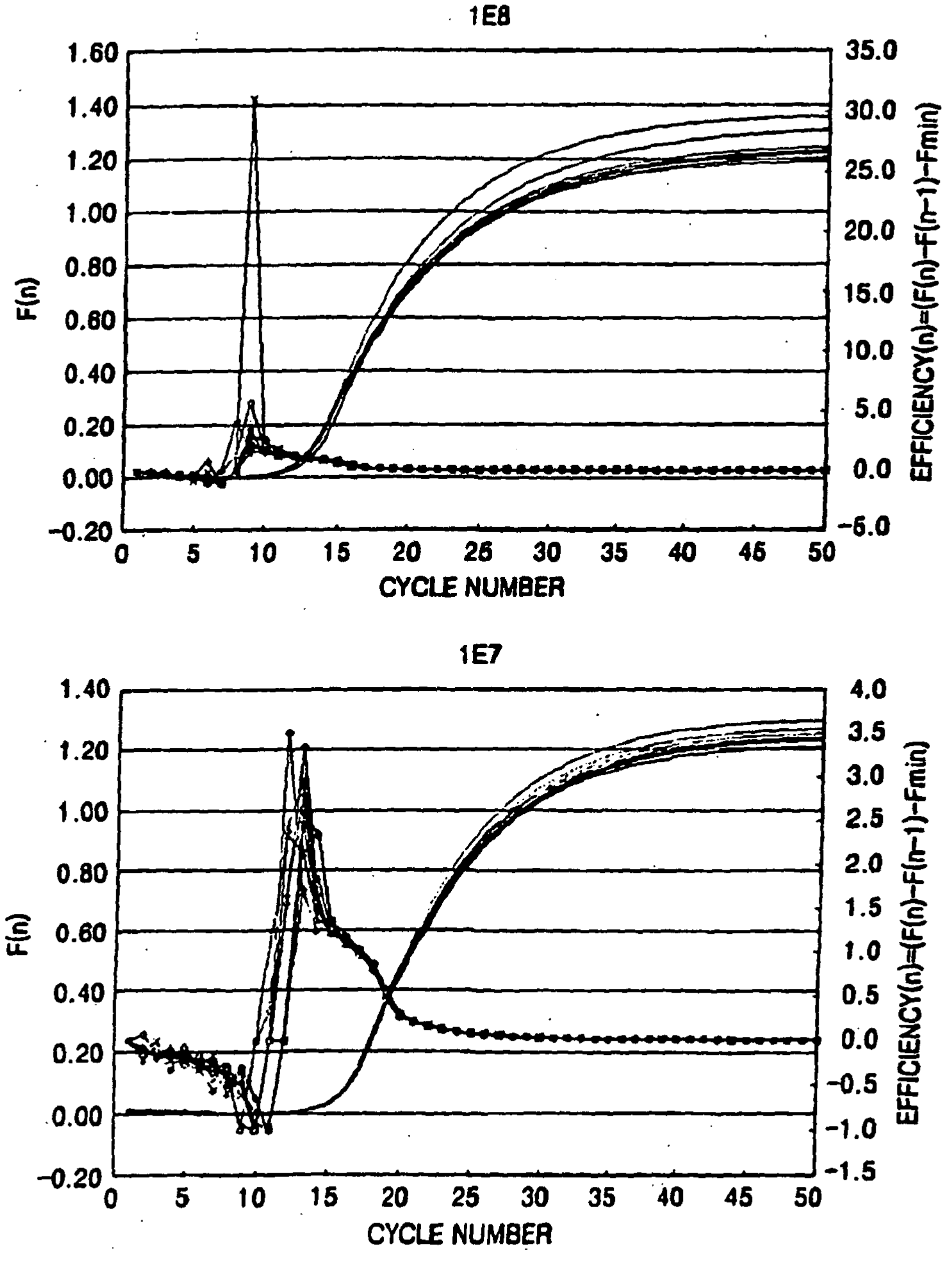
1E8
F(n)
1.60
1.40
1.20
1.00
0.80
0.60
0.40
0.20
0.00
-0.20
35.0
30.0
25.0
20.0
15.0
10.0
5.0
0.0
-5.0
EFFICIENCY(n)=(F(n)-F(n-1))-Fmin
0 5 10 15 20 25 30 35 40 45 50
CYCLE NUMBER
1E7
F(n)
1.40
1.20
1.00
0.80
0.60
0.40
0.20
0.00
-0.20
4.0
3.5
3.0
2.5
2.0
1.5
1.0
0.5
0.0
-0.5
-1.0
-1.5
EFFICIENCY(n)=(F(n)-F(n-1))-Fmin
0 5 10 15 20 25 30 35 40 45 50
CYCLE NUMBER

FIG. 8B

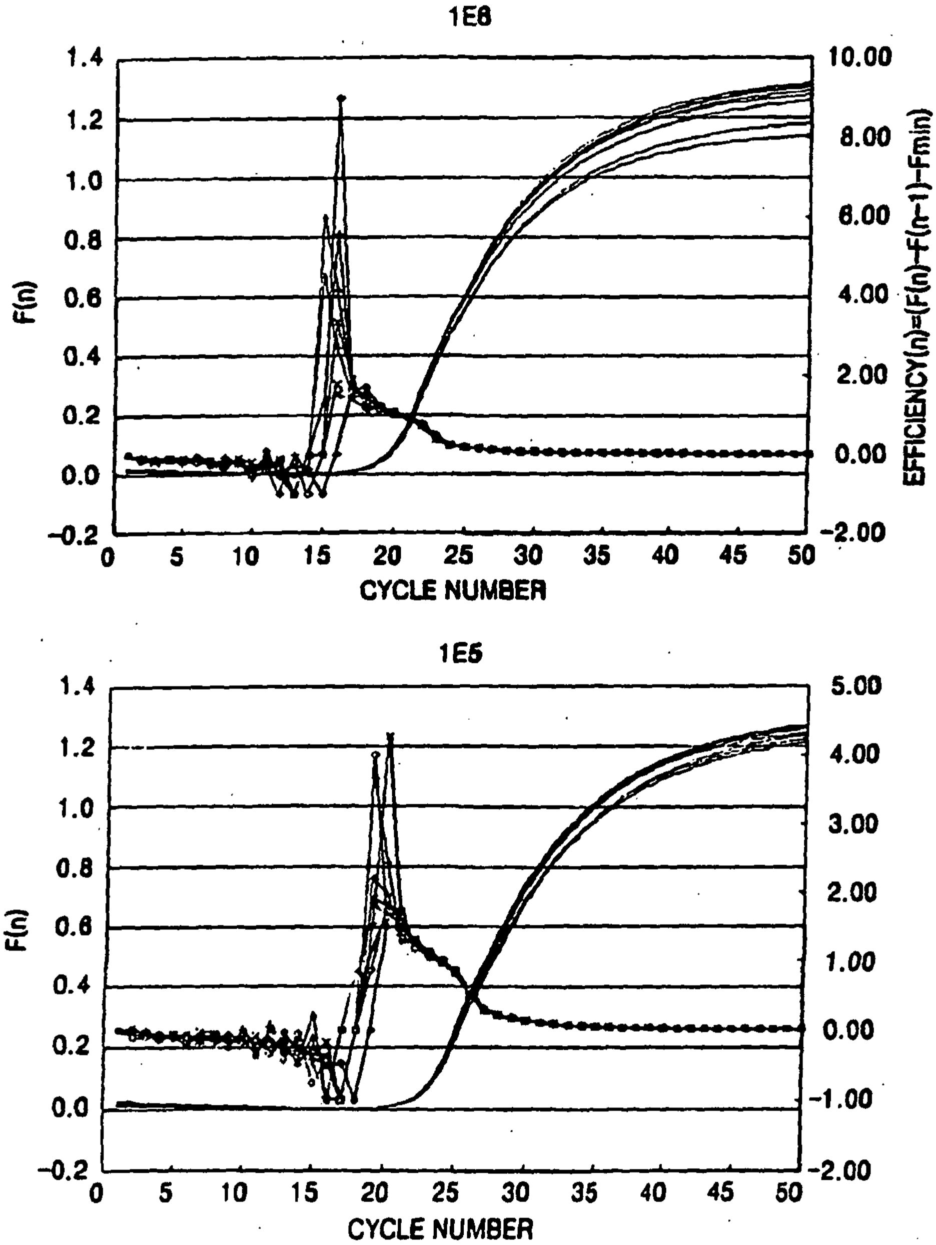
1E6
F(n)
EFFICIENCY(n)=(F(n)-F(n-1))-Fmin)
1.4
1.2
1.0
0.8
0.6
0.4
0.2
0.0
-0.2
10.00
8.00
6.00
4.00
2.00
0.00
-2.00
0 5 10 15 20 25 30 35 40 45 50
CYCLE NUMBER
1E5
F(n)
1.4
1.2
1.0
0.8
0.6
0.4
0.2
0.0
-0.2
5.00
4.00
3.00
2.00
1.00
0.00
-1.00
-2.00
0 5 10 15 20 25 30 35 40 45 50
CYCLE NUMBER

FIG. 8C

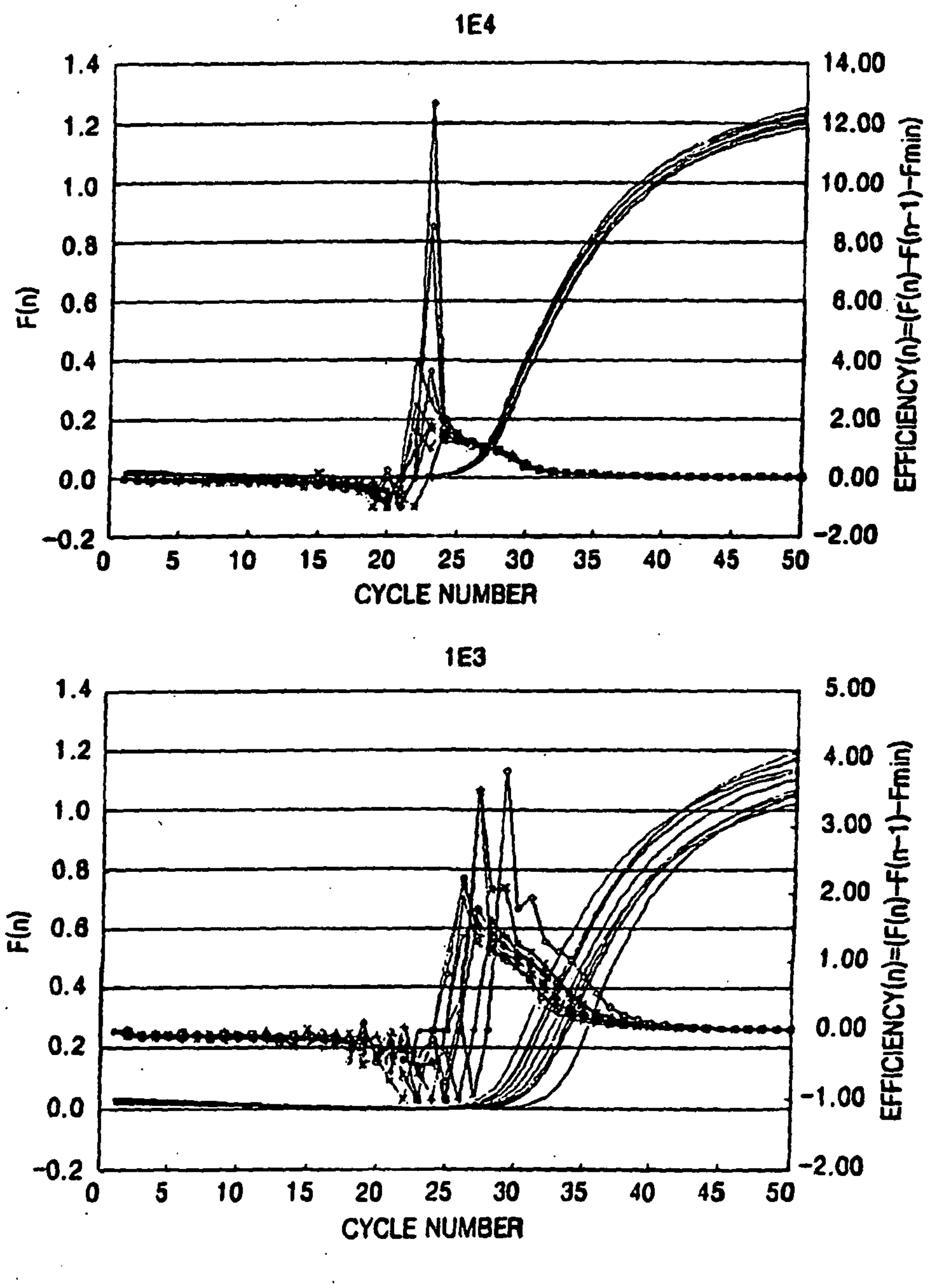
1E4
F(n)
EFFICIENCY(n)=(F(n)−F(n−1)−Fmin)
CYCLE NUMBER
1E3
F(n)
EFFICIENCY(n)=(F(n)−F(n−1)−Fmin)
CYCLE NUMBER

FIG. 8D

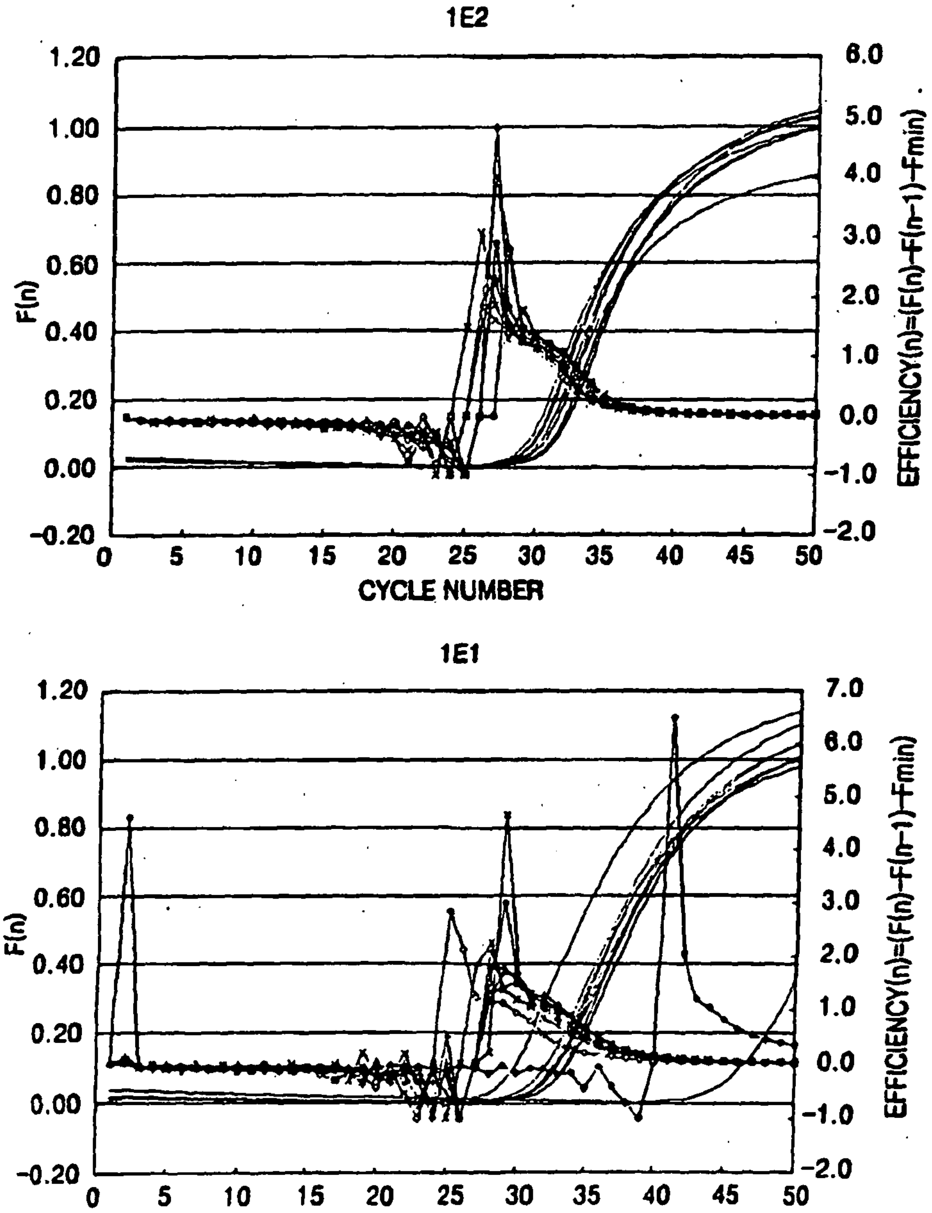
1E2
1.20
1.00
0.80
0.60
0.40
0.20
0.00
-0.20
F(n)
6.0
5.0
4.0
3.0
2.0
1.0
0.0
-1.0
-2.0
EFFICIENCY(n)=(F(n)-F(n-1))-Fmin)
0
5
10
15
20
25
30
35
40
45
50
CYCLE NUMBER
1E1
1.20
1.00
0.80
0.60
0.40
0.20
0.00
-0.20
F(n)
7.0
6.0
5.0
4.0
3.0
2.0
1.0
0.0
-1.0
-2.0
EFFICIENCY(n)=(F(n)-F(n-1))-Fmin)
0
5
10
15
20
25
30
35
40
45
50
CYCLE NUMBER

FIG. 8E

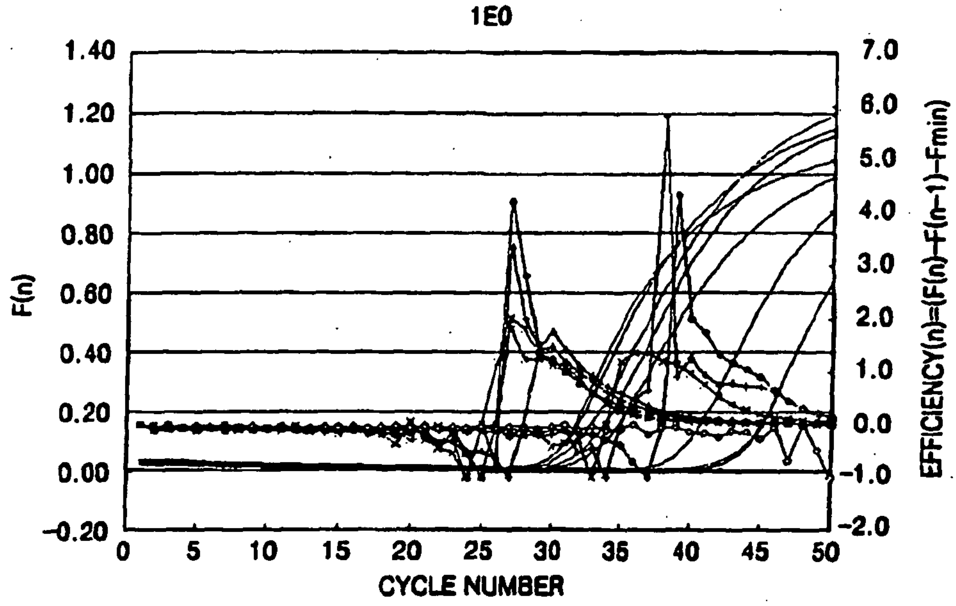
1E0
F(n)
EFFICIENCY(n)=(F(n)-F(n-1))-Fmin)
CYCLE NUMBER
1.40
1.20
1.00
0.80
0.60
0.40
0.20
0.00
-0.20
7.0
6.0
5.0
4.0
3.0
2.0
1.0
0.0
-1.0
-2.0
0
5
10
15
20
25
30
35
40
45
50

FIG. 9

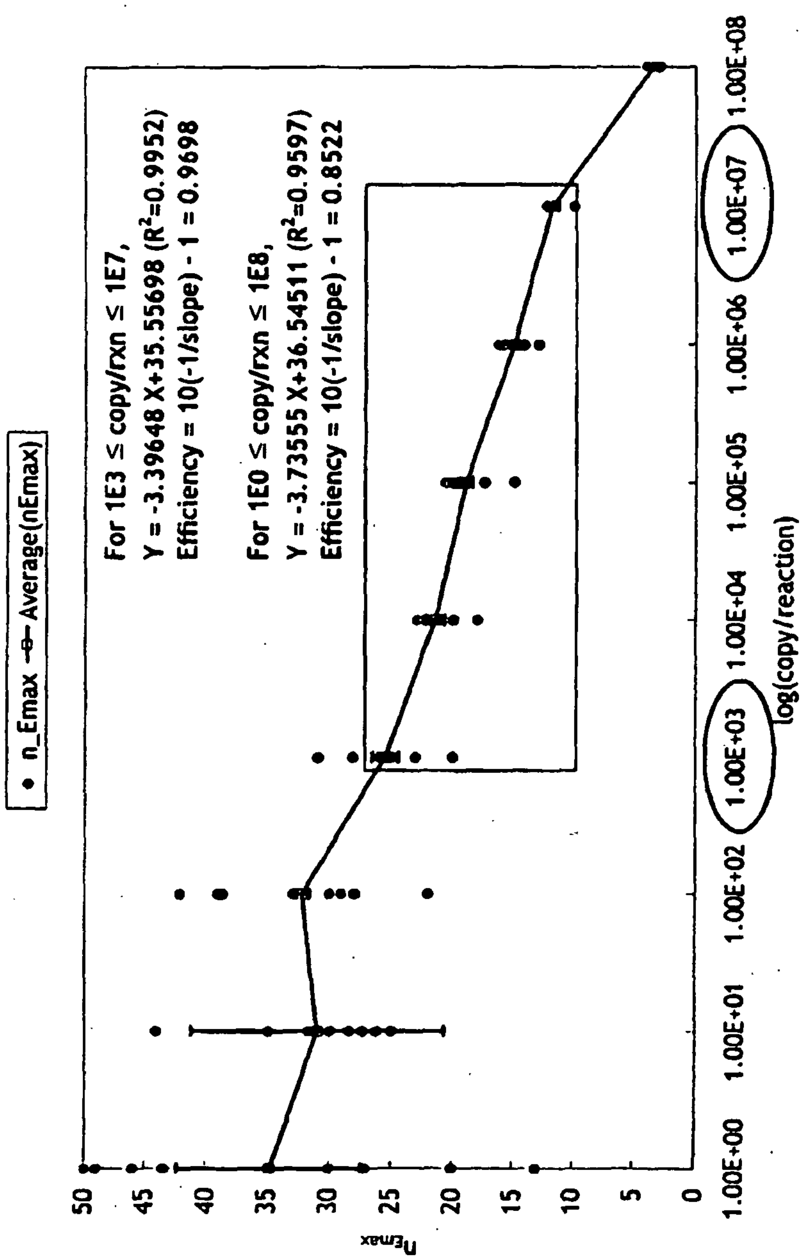
● n_Emax —□— Average(nEmax)
For 1E3 ≤ copy/rxn ≤ 1E7,
Y = -3.39648 X+35.55698 (R²=0.9952)
Efficiency = 10(-1/slope) - 1 = 0.9698
For 1E0 ≤ copy/rxn ≤ 1E8,
Y = -3.73555 X+36.54511 (R²=0.9597)
Efficiency = 10(-1/slope) - 1 = 0.8522
n_Emax
50
45
40
35
30
25
20
15
10
5
0
1.00E+00
1.00E+01
1.00E+02
1.00E+03
1.00E+04
1.00E+05
1.00E+06
1.00E+07
1.00E+08
log(copy/reaction)

FIG. 10

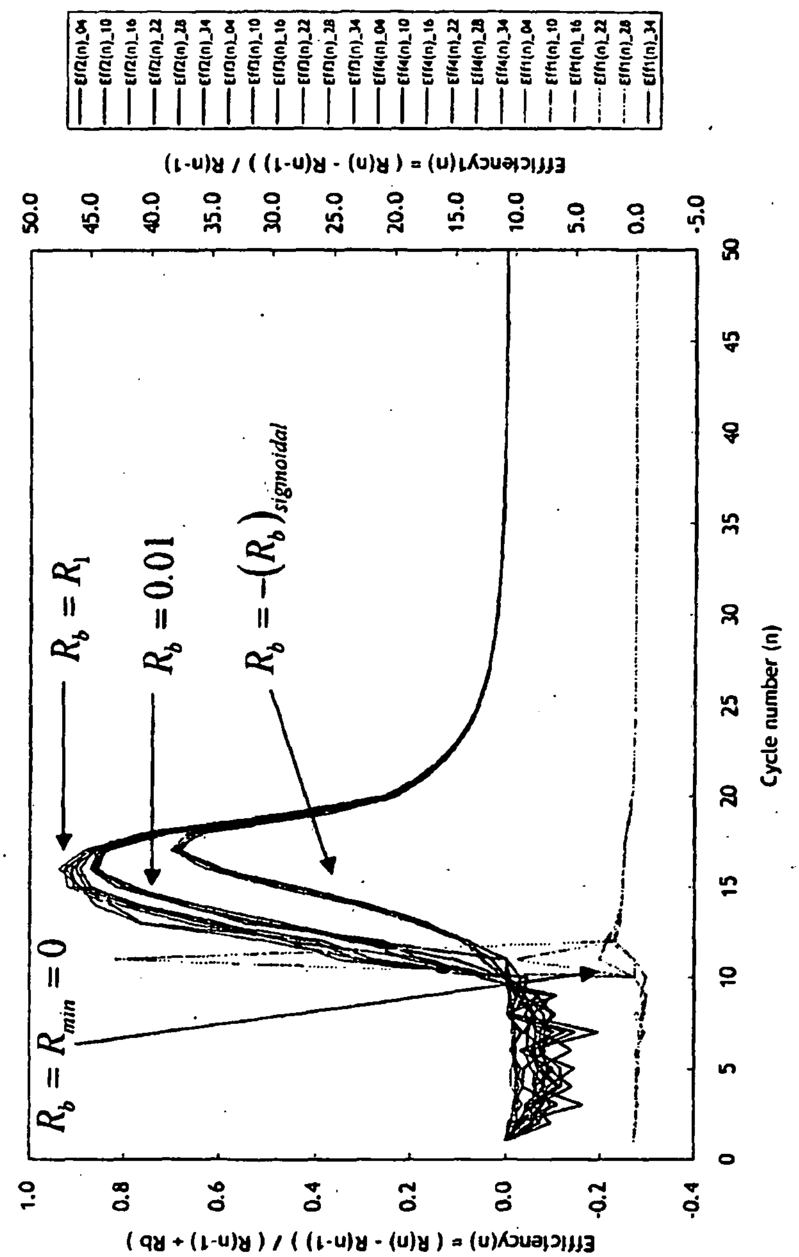

$R_b = R_{min} = 0$
$R_b = R_1$
$R_b = 0.01$
$R_b = -(R_b)_{sigmoidal}$
Efficiency(n) = ( R(n) - R(n-1) ) / ( R(n-1) + Rb )
Efficiency1(n) = ( R(n) - R(n-1) ) / R(n-1)
Cycle number (n)
Eff2(n)_04
Eff2(n)_10
Eff2(n)_16
Eff2(n)_22
Eff2(n)_28
Eff2(n)_34
Eff3(n)_04
Eff3(n)_10
Eff3(n)_16
Eff3(n)_22
Eff3(n)_28
Eff3(n)_34
Eff4(n)_04
Eff4(n)_10
Eff4(n)_16
Eff4(n)_22
Eff4(n)_28
Eff4(n)_34
Eff1(n)_04
Eff1(n)_10
Eff1(n)_16
Eff1(n)_22
Eff1(n)_28
Eff1(n)_34

FIG. 11A

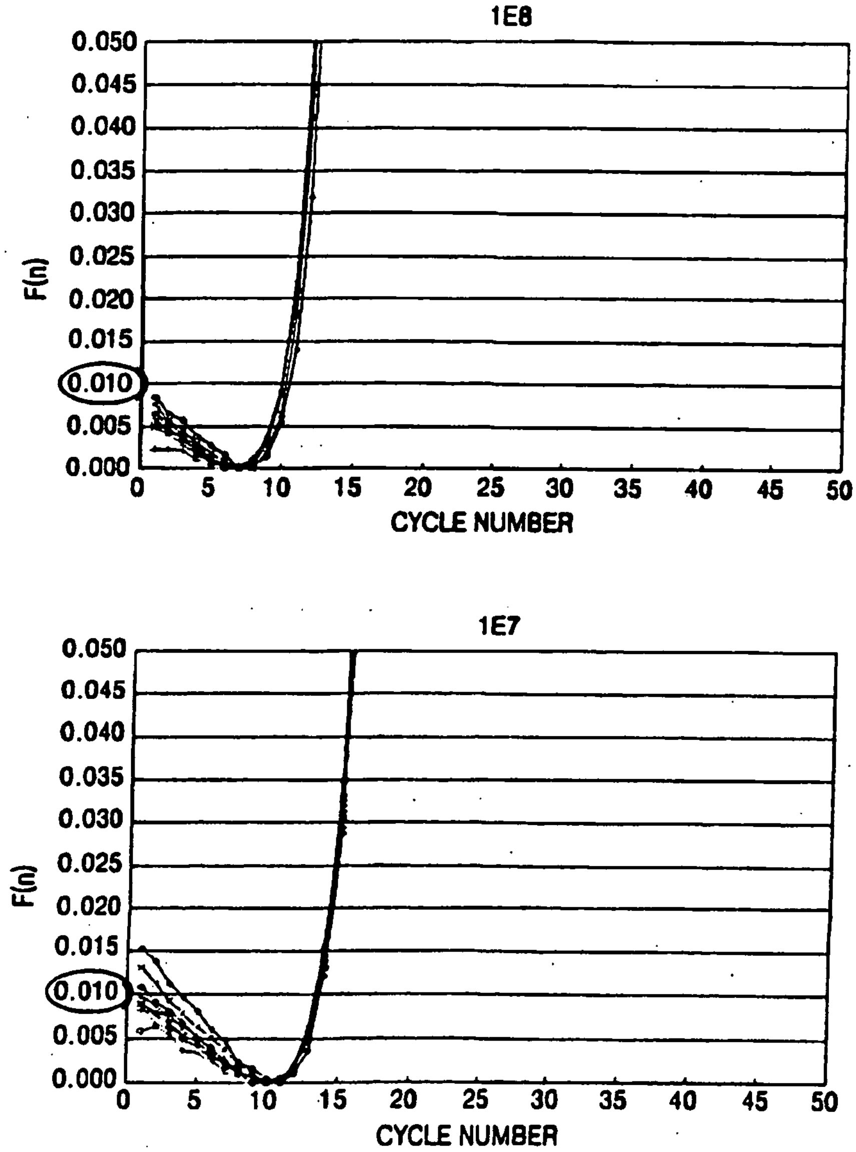
1E8
F(n)
0.050
0.045
0.040
0.035
0.030
0.025
0.020
0.015
0.010
0.005
0.000
0 5 10 15 20 25 30 35 40 45 50
CYCLE NUMBER
1E7
F(n)
0.050
0.045
0.040
0.035
0.030
0.025
0.020
0.015
0.010
0.005
0.000
0 5 10 15 20 25 30 35 40 45 50
CYCLE NUMBER

FIG. 11B

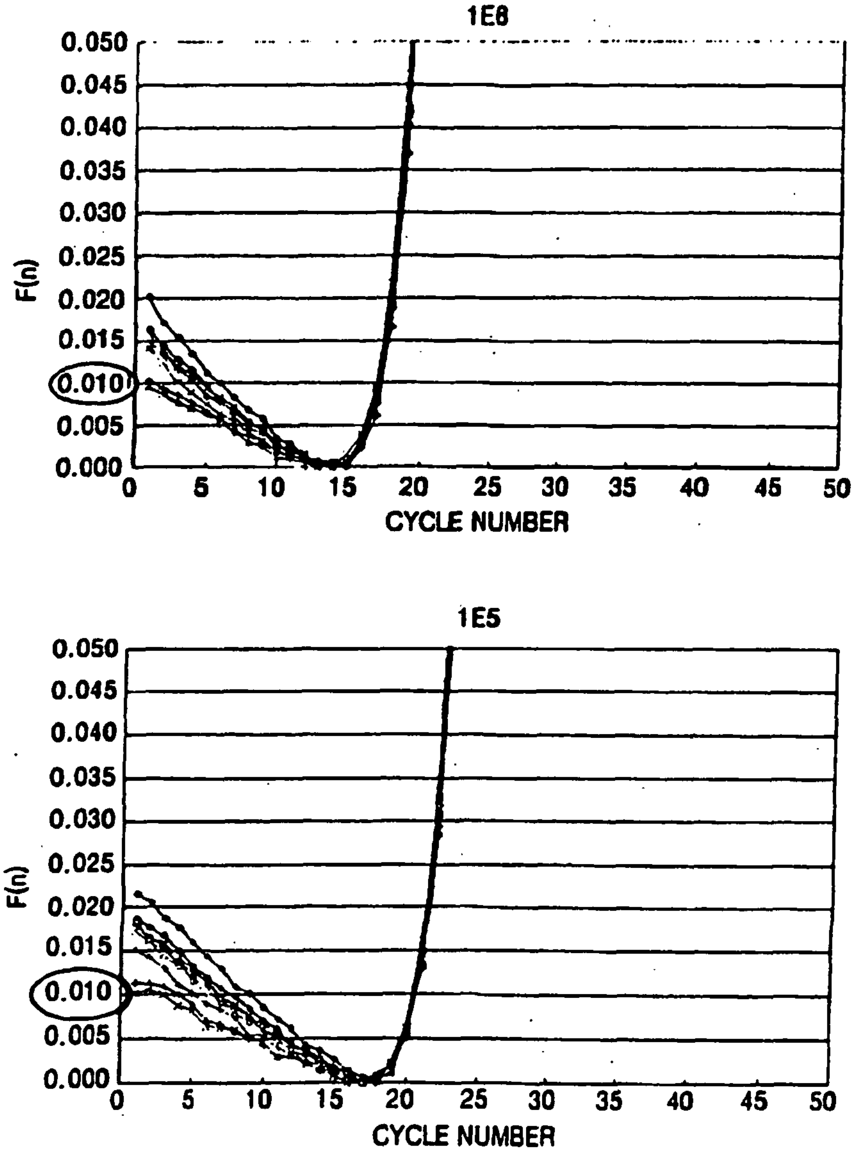
1E8
0.050
0.045
0.040
0.035
0.030
0.025
0.020
0.015
0.010
0.005
0.000
F(n)
0 5 10 15 20 25 30 35 40 45 50
CYCLE NUMBER
1E5
0.050
0.045
0.040
0.035
0.030
0.025
0.020
0.015
0.010
0.005
0.000
F(n)
0 5 10 15 20 25 30 35 40 45 50
CYCLE NUMBER

FIG. 11C

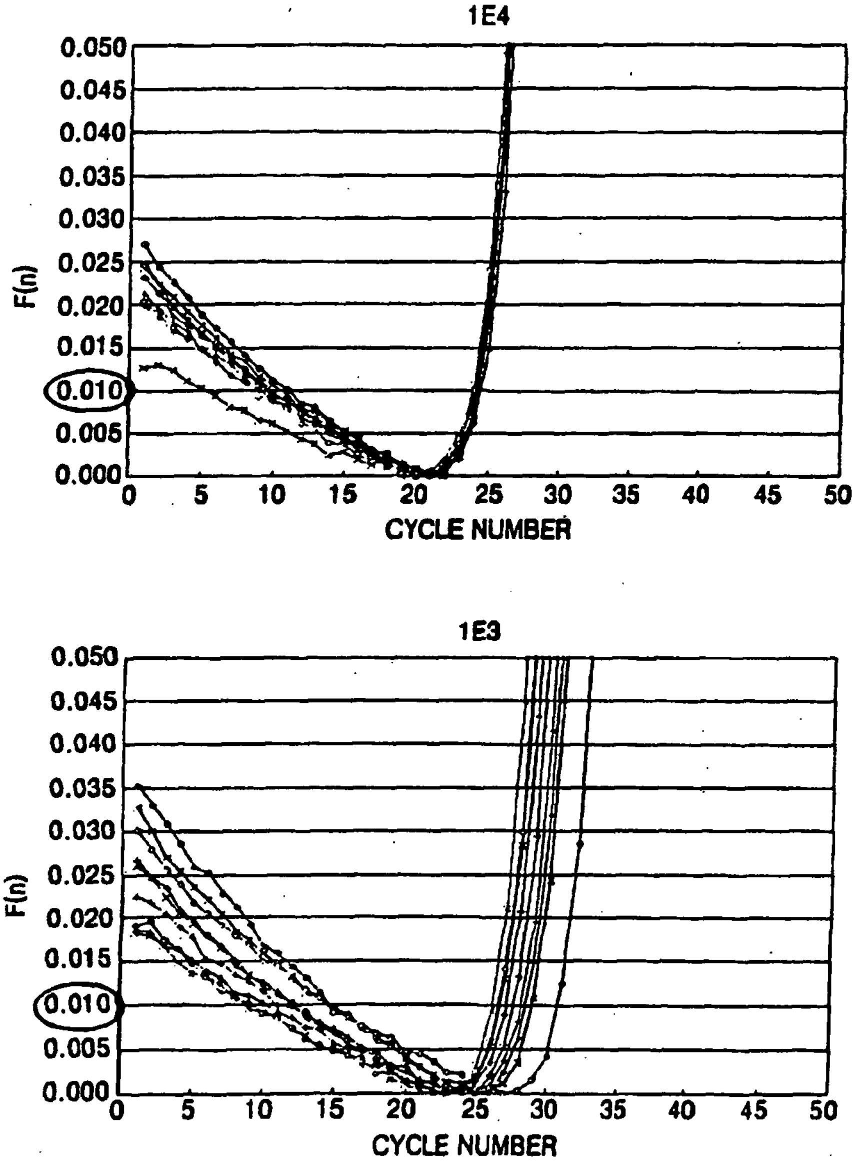
1E4
F(n)
0.050
0.045
0.040
0.035
0.030
0.025
0.020
0.015
0.010
0.005
0.000
0
5
10
15
20
25
30
35
40
45
50
CYCLE NUMBER
1E3
F(n)
0.050
0.045
0.040
0.035
0.030
0.025
0.020
0.015
0.010
0.005
0.000
0
5
10
15
20
25
30
35
40
45
50
CYCLE NUMBER

FIG. 11D

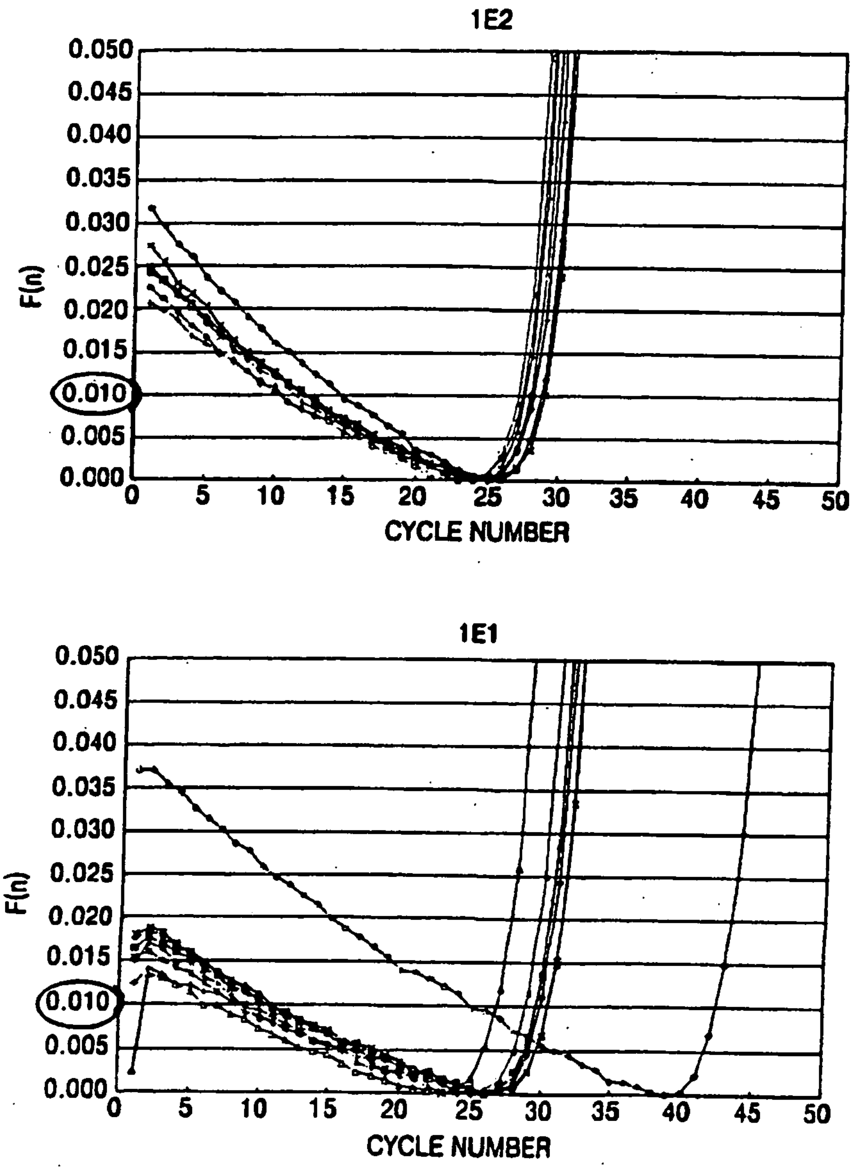
1E2
F(n)
0.050
0.045
0.040
0.035
0.030
0.025
0.020
0.015
0.010
0.005
0.000
0 5 10 15 20 25 30 35 40 45 50
CYCLE NUMBER
1E1
F(n)
0.050
0.045
0.040
0.035
0.030
0.025
0.020
0.015
0.010
0.005
0.000
0 5 10 15 20 25 30 35 40 45 50
CYCLE NUMBER

FIG. 11E

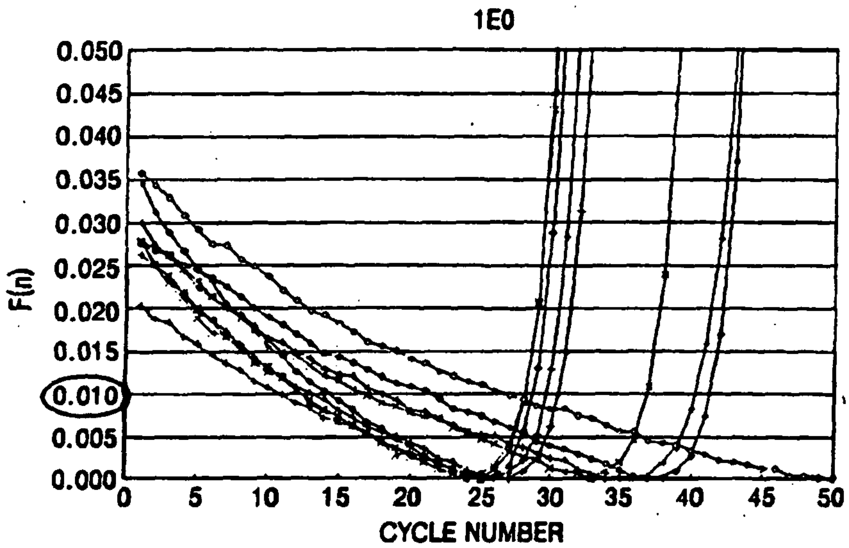
1E0
0.050
0.045
0.040
0.035
0.030
0.025
0.020
0.015
0.010
0.005
0.000
F(n)
0
5
10
15
20
25
30
35
40
45
50
CYCLE NUMBER

FIG. 12A

$$(R_n + R_b) = (1 + E_n)(R_{n-1} + R_b)$$

$$E_n = \frac{R_n - R_{n-1}}{R_{n-1} + R_b} \rightarrow R_b > 0$$

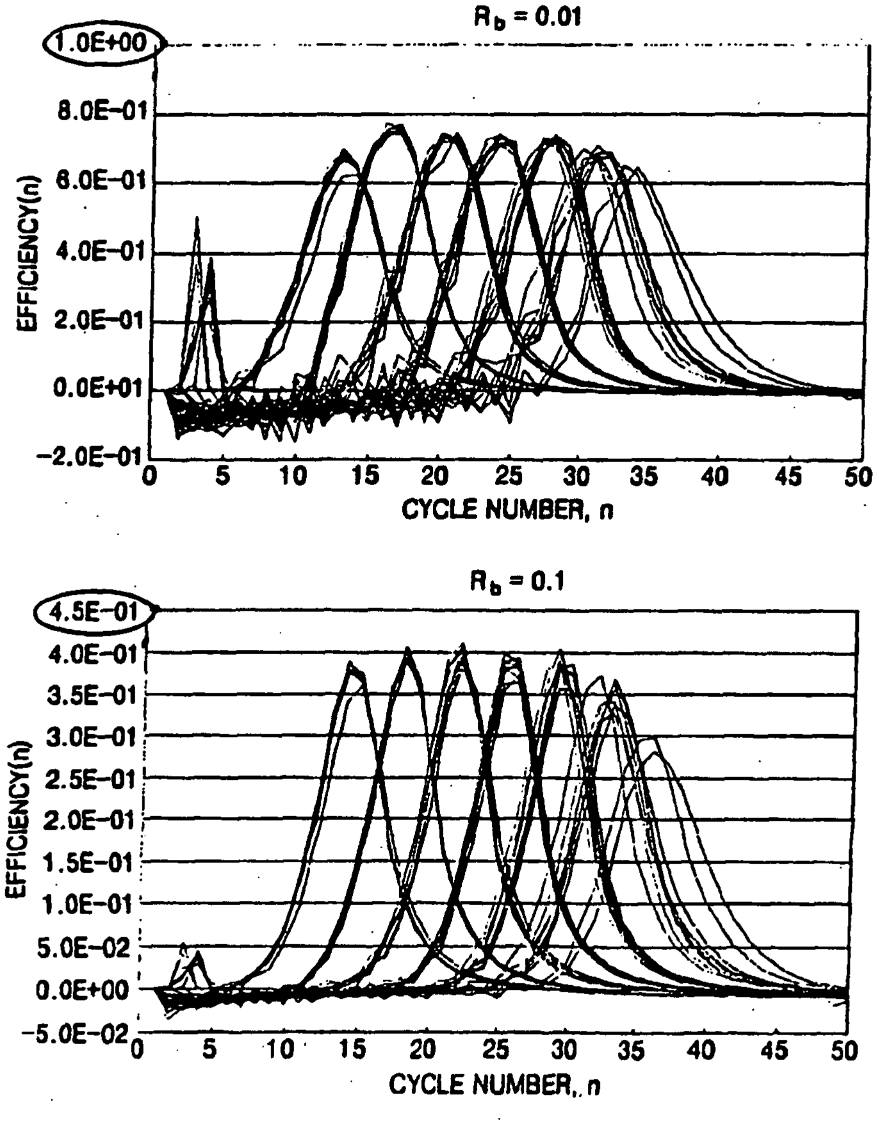

FIG. 12B

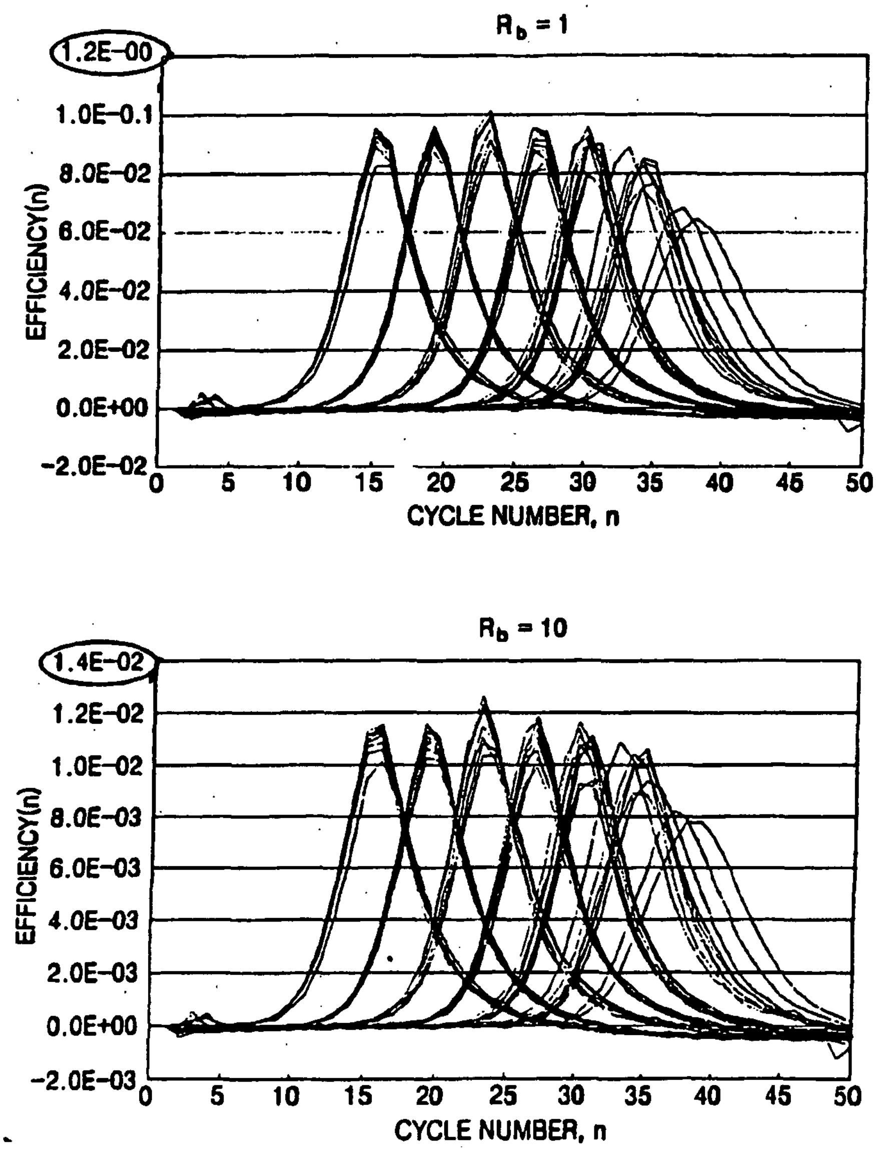

Rb = 1
1.2E-00
1.0E-0.1
8.0E-02
6.0E-02
4.0E-02
2.0E-02
0.0E+00
-2.0E-02
EFFICIENCY(n)
0 5 10 15 20 25 30 35 40 45 50
CYCLE NUMBER, n
Rb = 10
1.4E-02
1.2E-02
1.0E-02
8.0E-03
6.0E-03
4.0E-03
2.0E-03
0.0E+00
-2.0E-03
EFFICIENCY(n)
0 5 10 15 20 25 30 35 40 45 50
CYCLE NUMBER, n

FIG. 12C

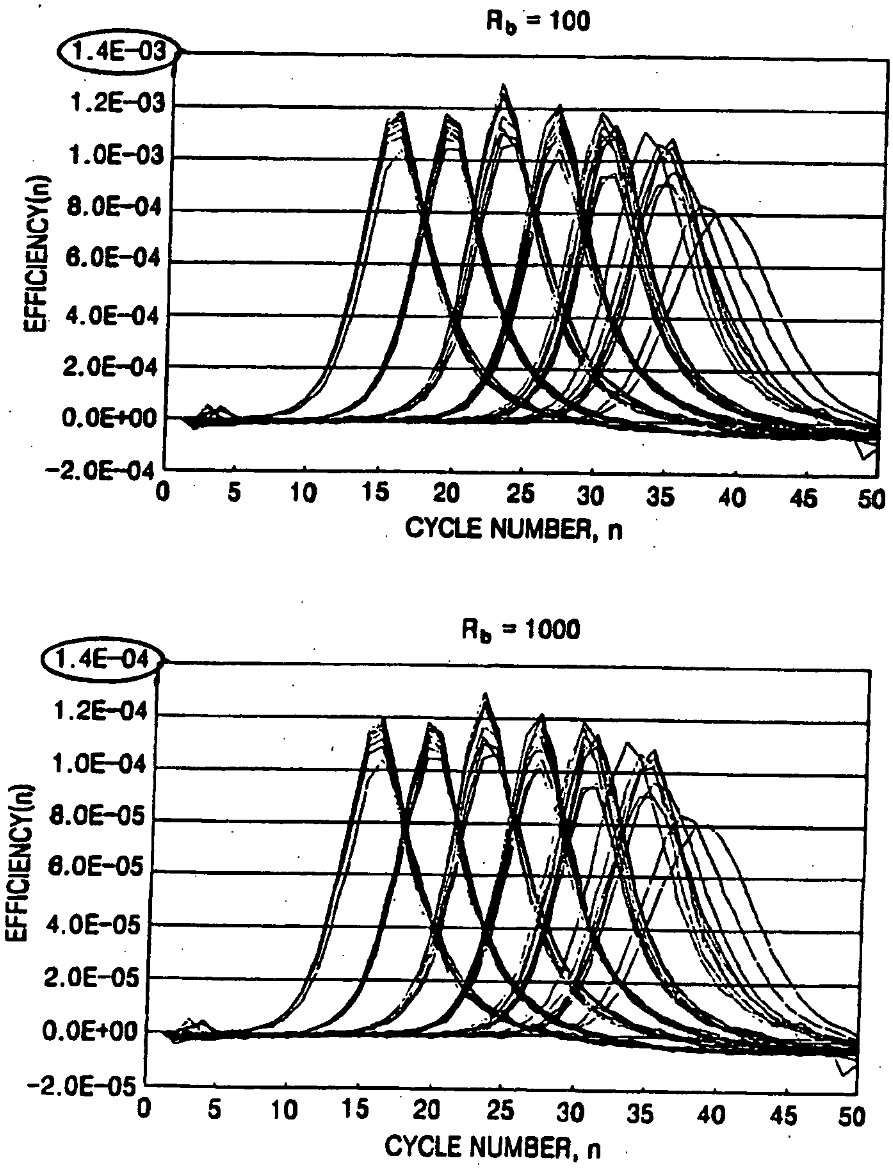
$R_b$ = 100
1.4E-03
1.2E-03
1.0E-03
8.0E-04
6.0E-04
4.0E-04
2.0E-04
0.0E+00
-2.0E-04
EFFICIENCY(n)
0 5 10 15 20 25 30 35 40 45 50
CYCLE NUMBER, n
$R_b$ = 1000
1.4E-04
1.2E-04
1.0E-04
8.0E-05
6.0E-05
4.0E-05
2.0E-05
0.0E+00
-2.0E-05
EFFICIENCY(n)
0 5 10 15 20 25 30 35 40 45 50
CYCLE NUMBER, n

FIG. 12D

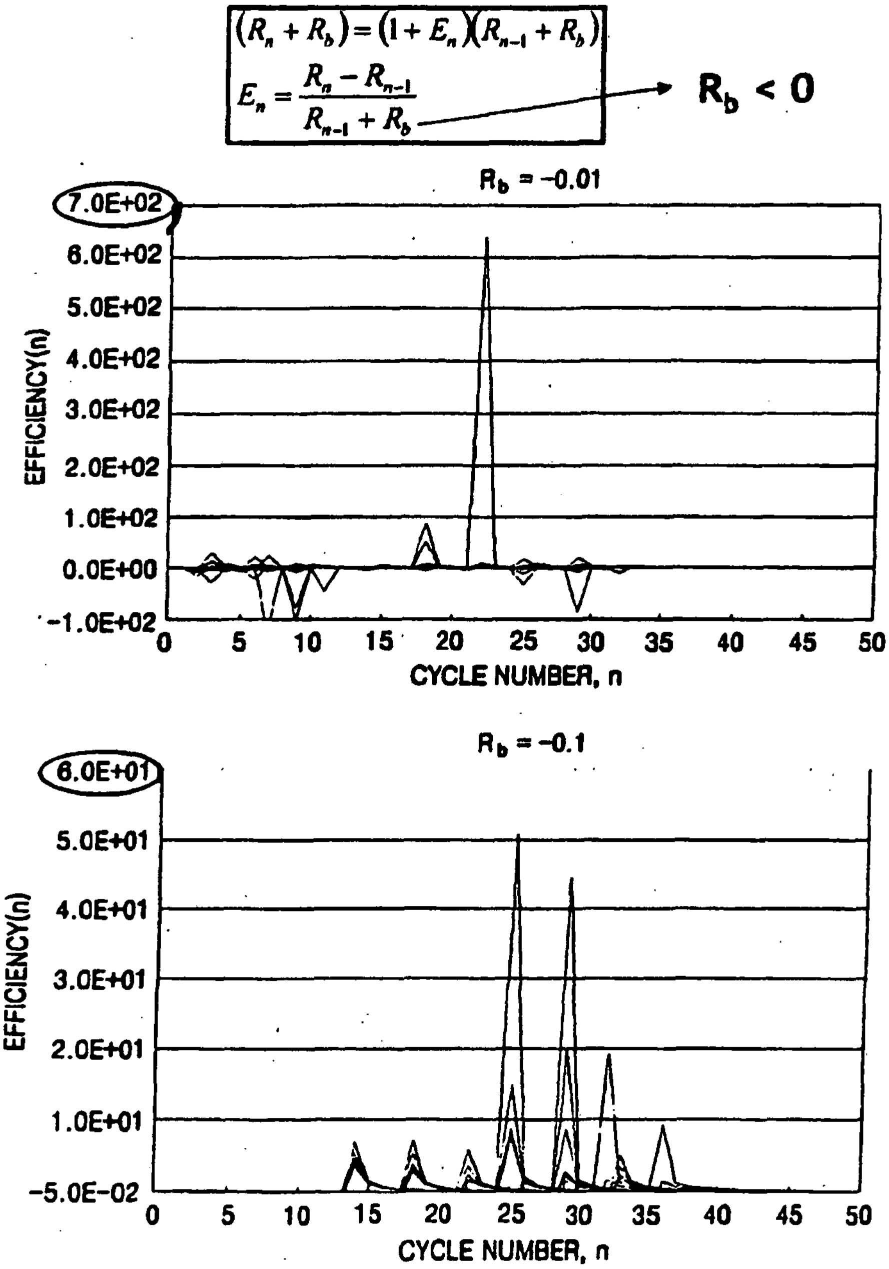
$(R_n + R_b) = (1 + E_n)(R_{n-1} + R_b)$
$E_n = \frac{R_n - R_{n-1}}{R_{n-1} + R_b}$
$R_b < 0$
$R_b = -0.01$
7.0E+02
6.0E+02
5.0E+02
4.0E+02
3.0E+02
2.0E+02
1.0E+02
0.0E+00
-1.0E+02
EFFICIENCY(n)
0 5 10 15 20 25 30 35 40 45 50
CYCLE NUMBER, n
$R_b = -0.1$
6.0E+01
5.0E+01
4.0E+01
3.0E+01
2.0E+01
1.0E+01
-5.0E-02
EFFICIENCY(n)
0 5 10 15 20 25 30 35 40 45 50
CYCLE NUMBER, n

FIG. 12E

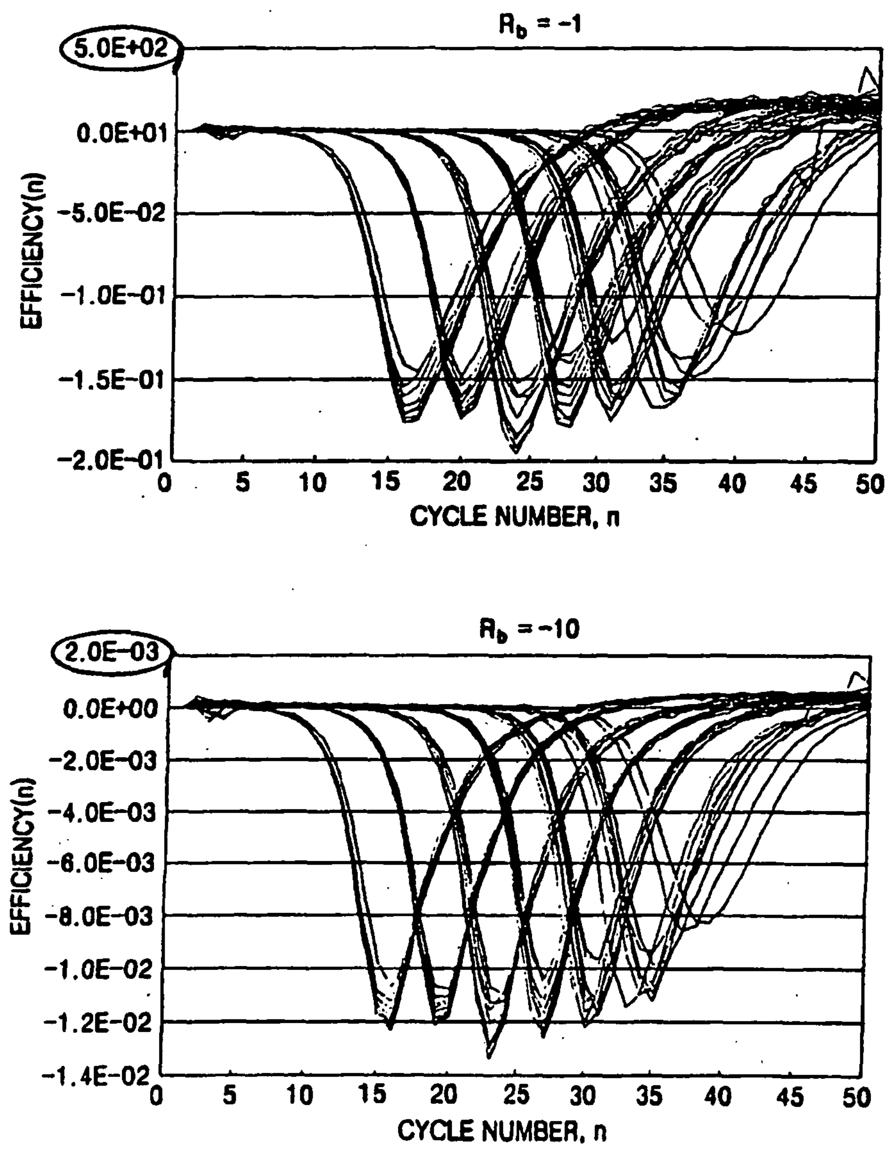
$R_b$ = -1
5.0E+02
0.0E+01
-5.0E-02
-1.0E-01
-1.5E-01
-2.0E-01
EFFICIENCY(n)
0 5 10 15 20 25 30 35 40 45 50
CYCLE NUMBER, n
$R_b$ = -10
2.0E-03
0.0E+00
-2.0E-03
-4.0E-03
-6.0E-03
-8.0E-03
-1.0E-02
-1.2E-02
-1.4E-02
EFFICIENCY(n)
0 5 10 15 20 25 30 35 40 45 50
CYCLE NUMBER, n

FIG. 12F

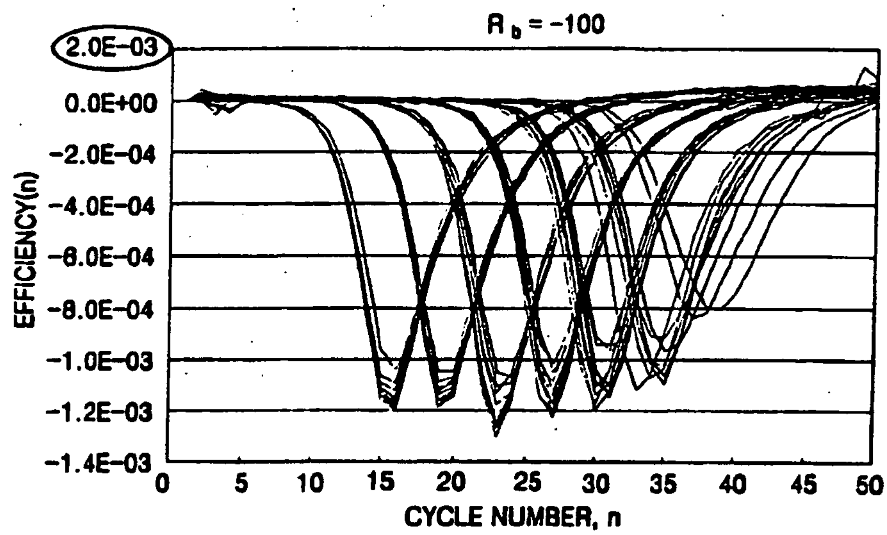
$R_b$ = -100
2.0E-03
0.0E+00
-2.0E-04
-4.0E-04
-6.0E-04
-8.0E-04
-1.0E-03
-1.2E-03
-1.4E-03
EFFICIENCY(n)
0 5 10 15 20 25 30 35 40 45 50
CYCLE NUMBER, n

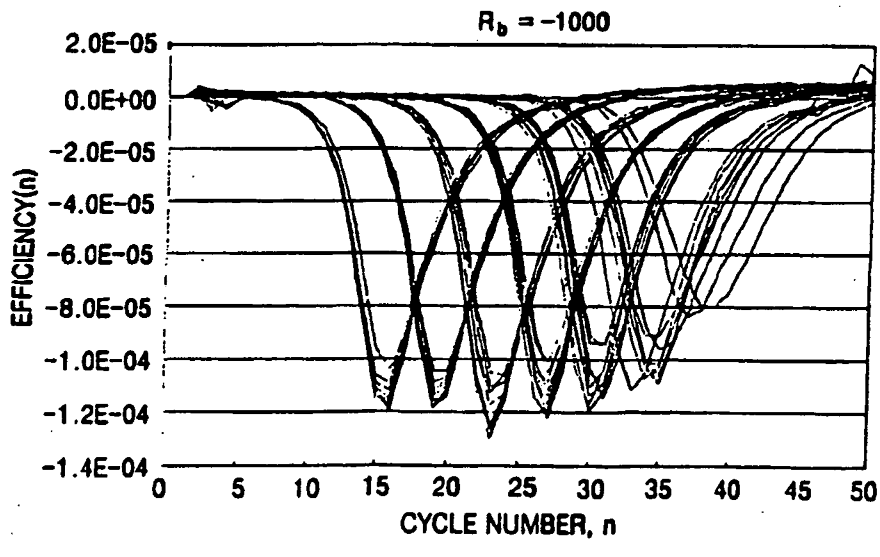
$R_b$ = -1000
2.0E-05
0.0E+00
-2.0E-05
-4.0E-05
-6.0E-05
-8.0E-05
-1.0E-04
-1.2E-04
-1.4E-04
EFFICIENCY(n)
0 5 10 15 20 25 30 35 40 45 50
CYCLE NUMBER, n

FIG. 12G

$R_b > 0$

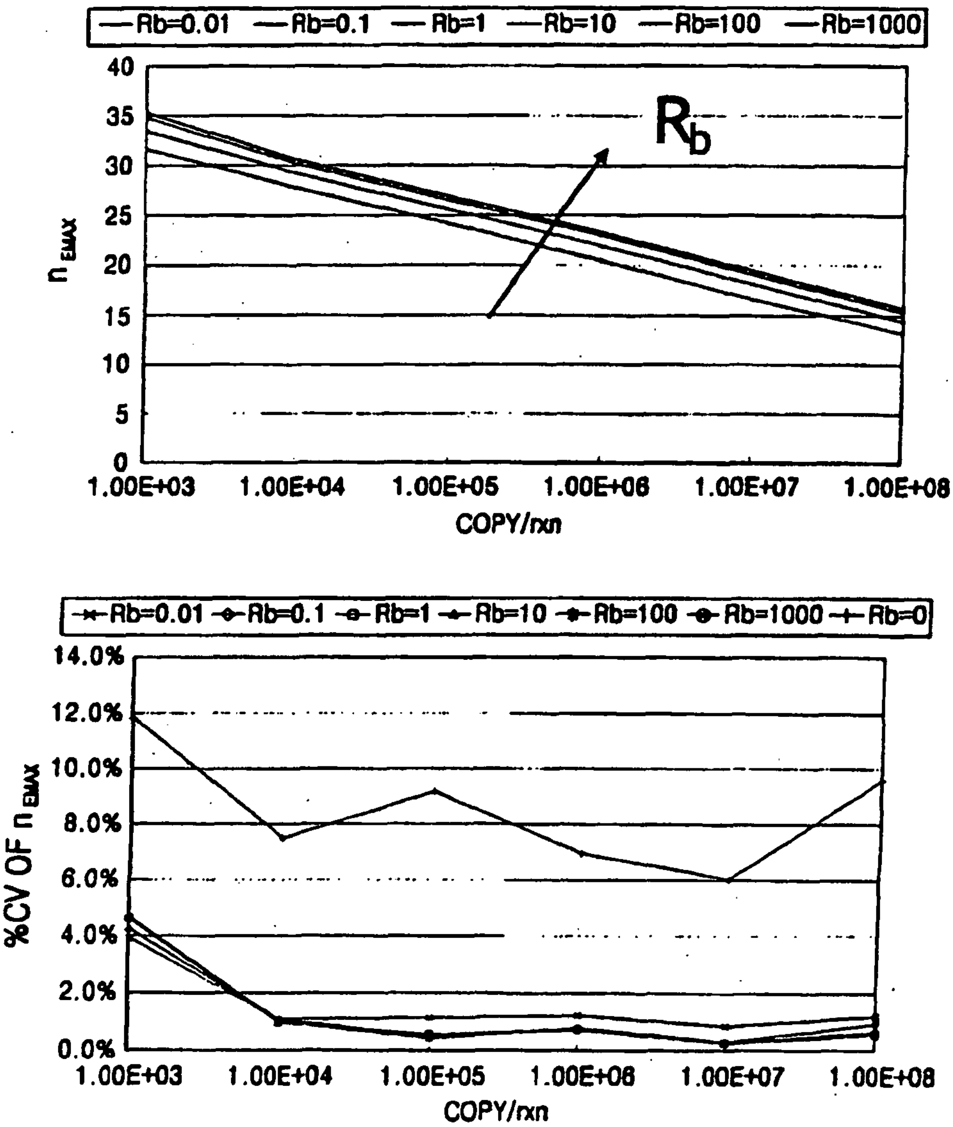

Rb=0.01
Rb=0.1
Rb=1
Rb=10
Rb=100
Rb=1000
40
35
30
25
20
15
10
5
0
n EMAX
Rb
1.00E+03
1.00E+04
1.00E+05
1.00E+06
1.00E+07
1.00E+08
COPY/rxn
Rb=0.01
Rb=0.1
Rb=1
Rb=10
Rb=100
Rb=1000
Rb=0
14.0%
12.0%
10.0%
8.0%
6.0%
4.0%
2.0%
0.0%
%CV OF n EMAX
1.00E+03
1.00E+04
1.00E+05
1.00E+06
1.00E+07
1.00E+08
COPY/rxn

FIG. 12H

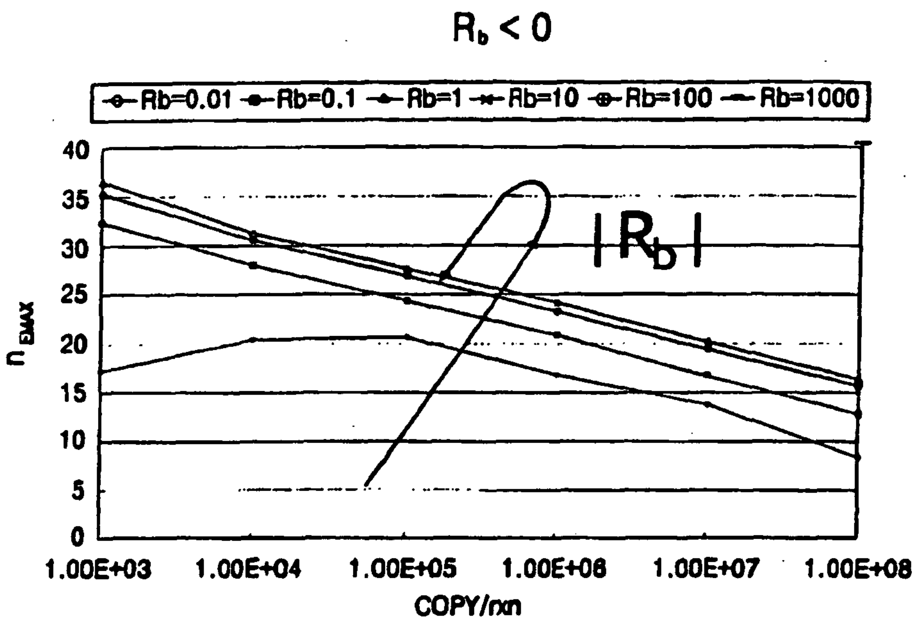
$R_b < 0$
Rb=0.01
Rb=0.1
Rb=1
Rb=10
Rb=100
Rb=1000
|Rb|
$n_{EMAX}$
40
35
30
25
20
15
10
5
0
1.00E+03
1.00E+04
1.00E+05
1.00E+06
1.00E+07
1.00E+08
COPY/rxn

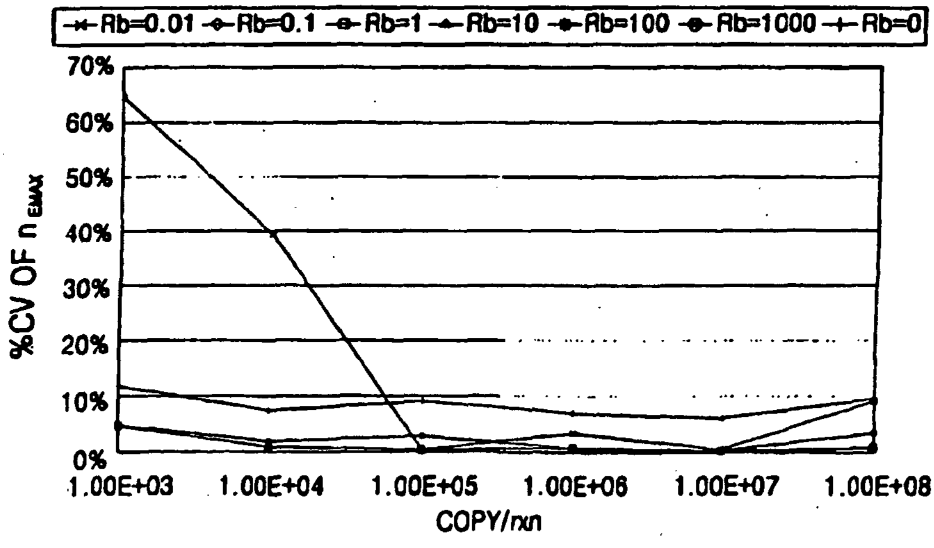
Rb=0.01
Rb=0.1
Rb=1
Rb=10
Rb=100
Rb=1000
Rb=0
%CV OF $n_{EMAX}$
70%
60%
50%
40%
30%
20%
10%
0%
1.00E+03
1.00E+04
1.00E+05
1.00E+06
1.00E+07
1.00E+08
COPY/rxn

FIG. 13

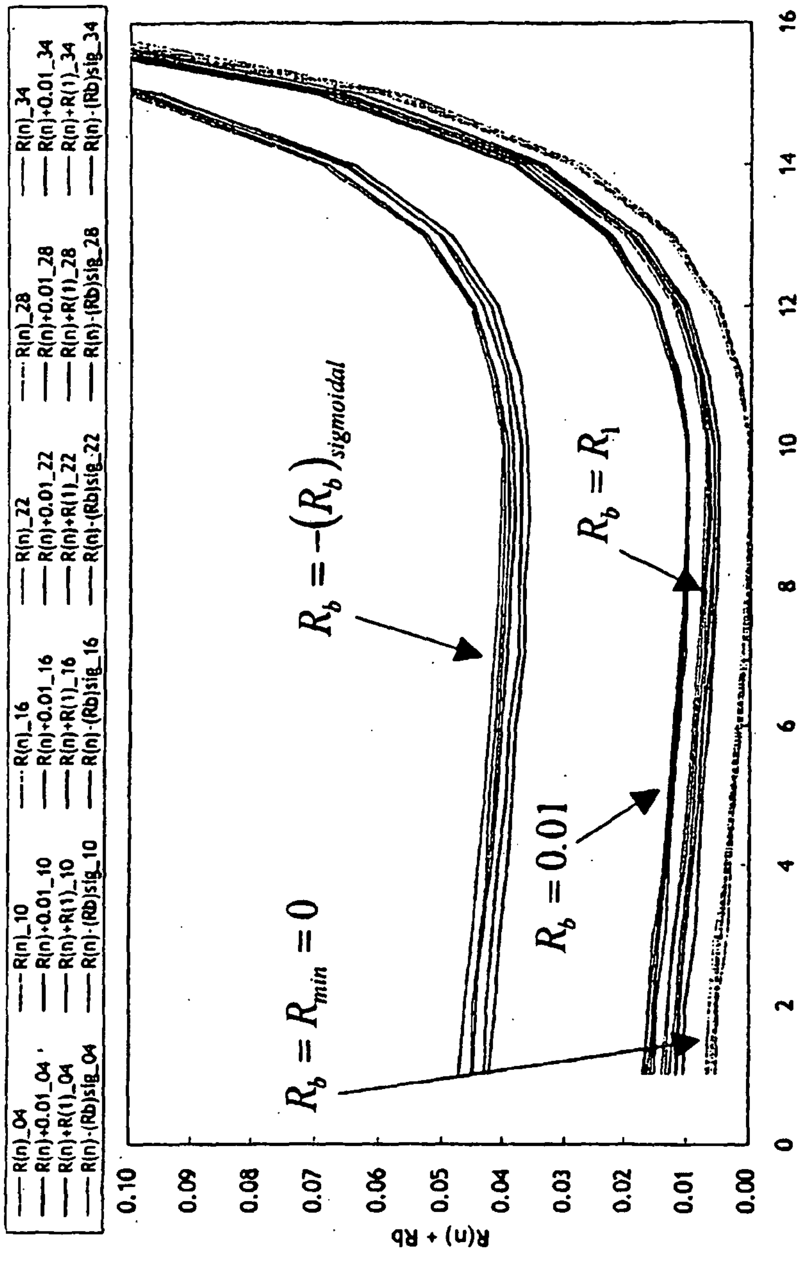
R(n)_04
R(n)+0.01_04
R(n)+R(1)_04
R(n)-(Rb)sig_04
R(n)_10
R(n)+0.01_10
R(n)+R(1)_10
R(n)-(Rb)sig_10
R(n)_16
R(n)+0.01_16
R(n)+R(1)_16
R(n)-(Rb)sig_16
R(n)_22
R(n)+0.01_22
R(n)+R(1)_22
R(n)-(Rb)sig_22
R(n)_28
R(n)+0.01_28
R(n)+R(1)_28
R(n)-(Rb)sig_28
R(n)_34
R(n)+0.01_34
R(n)+R(1)_34
R(n)-(Rb)sig_34
R(n) + Rb
0.10
0.09
0.08
0.07
0.06
0.05
0.04
0.03
0.02
0.01
0.00
0
2
4
6
8
10
12
14
16
Cycle number (n)
$R_b = R_{min} = 0$
$R_b = 0.01$
$R_b = -(R_b)_{sigmoidal}$
$R_b = R_1$

FIG. 14A

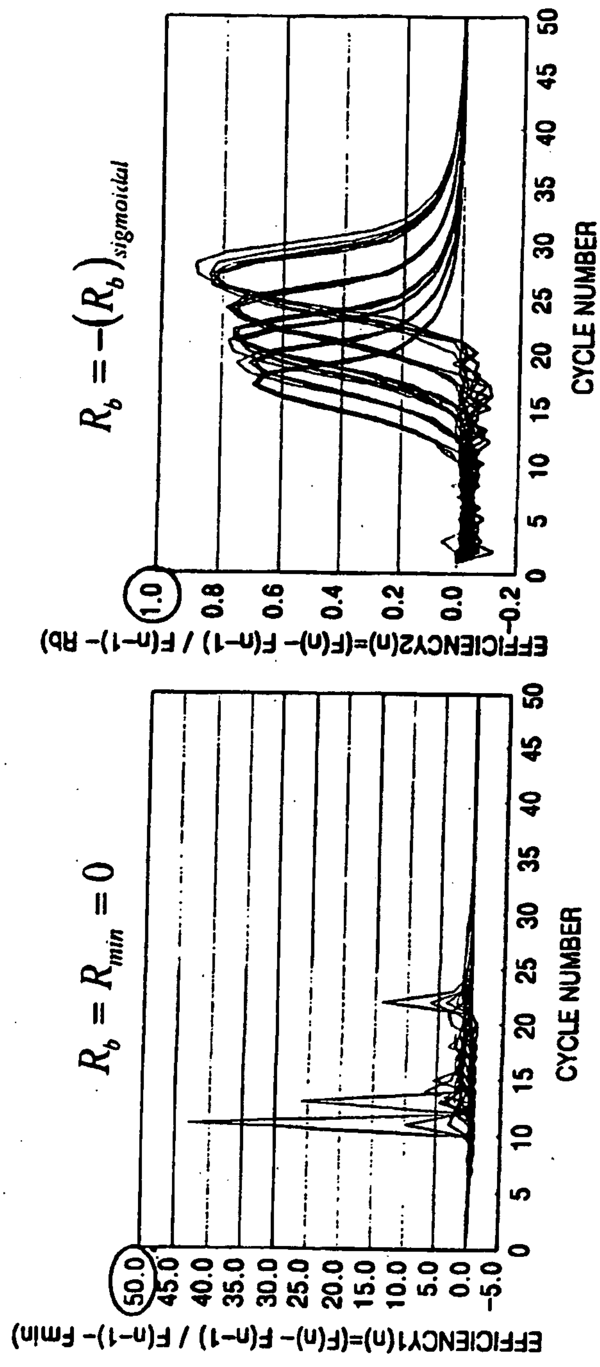
$R_b = R_{min} = 0$
EFFICIENCY1(n)=(F(n)- F(n-1) / F(n-1)- Fmin)
50.0
45.0
40.0
35.0
30.0
25.0
20.0
15.0
10.0
5.0
0.0
-5.0
0
5
10
15
20
25
30
35
40
45
50
CYCLE NUMBER
$R_b = -(R_b)_{sigmoidal}$
EFFICIENCY2(n)=(F(n)- F(n-1) / F(n-1)- Rb)
1.0
0.8
0.6
0.4
0.2
0.0
-0.2
0
5
10
15
20
25
30
35
40
45
50
CYCLE NUMBER

FIG. 14B

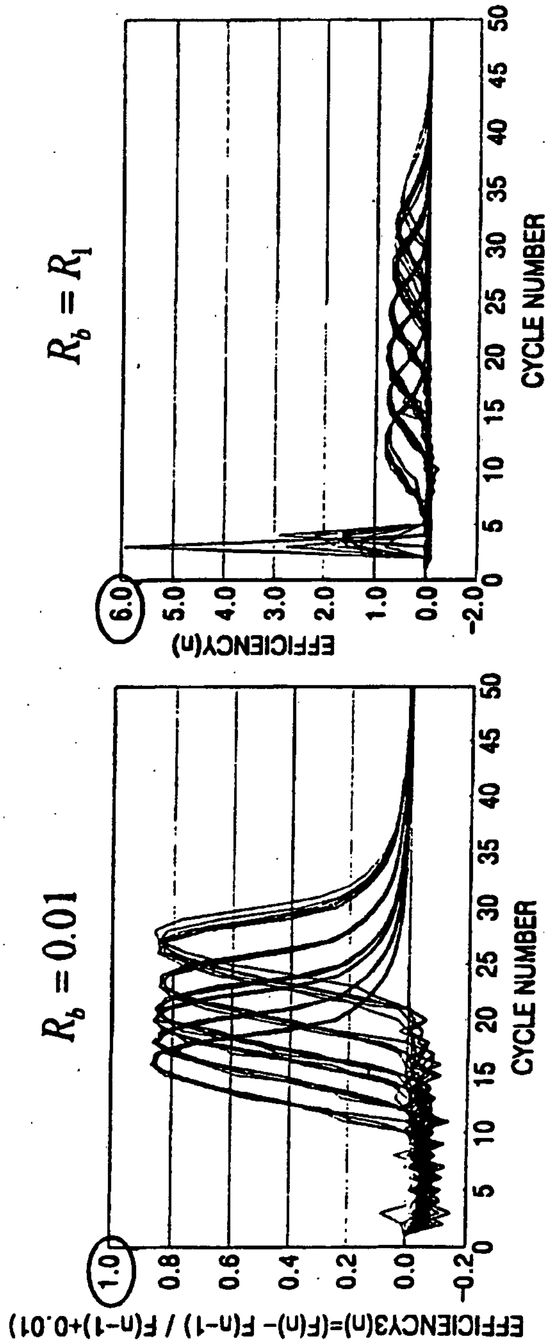
$R_b = 0.01$
EFFICIENCY3(n)=(F(n)− F(n−1) / F(n−1)+0.01)
1.0
0.8
0.6
0.4
0.2
0.0
−0.2
0
5
10
15
20
25
30
35
40
45
50
CYCLE NUMBER
$R_b = R_1$
EFFICIENCY(n)
6.0
5.0
4.0
3.0
2.0
1.0
0.0
−2.0
CYCLE NUMBER

FIG. 15

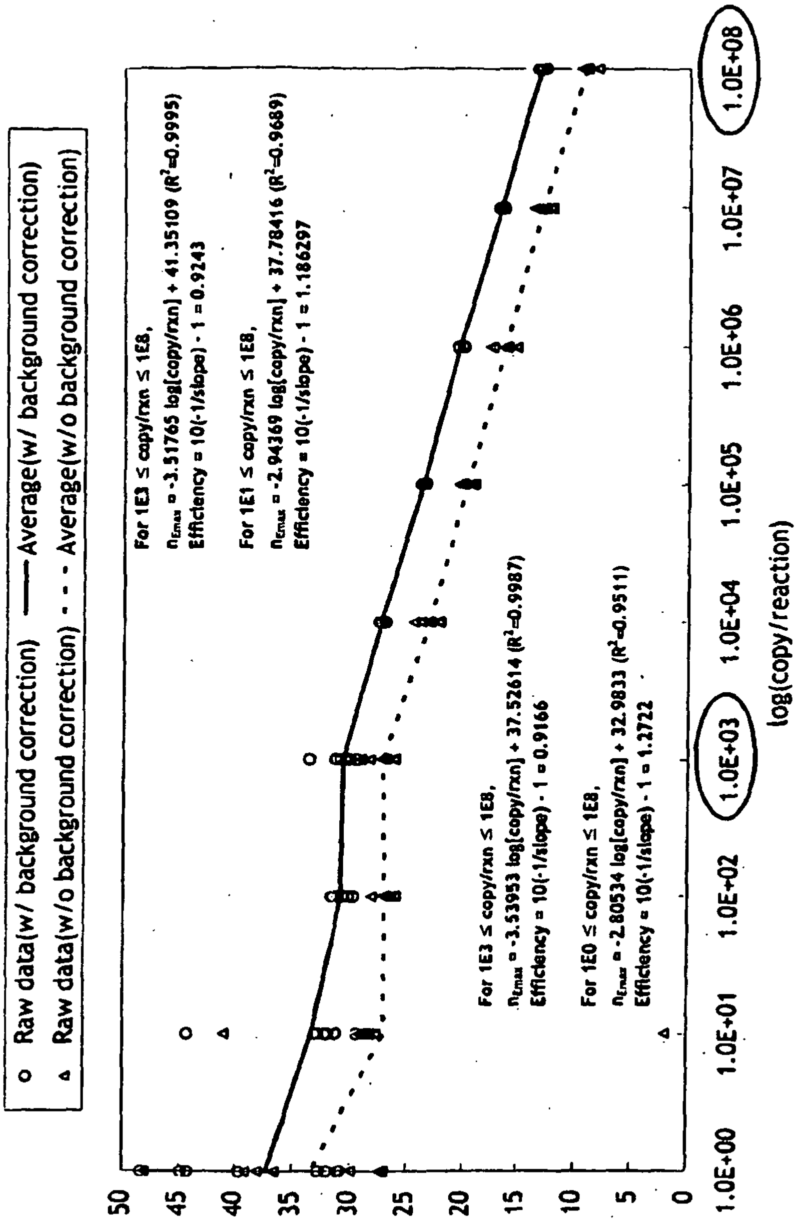

Raw data(w/ background correction)
Average(w/ background correction)
Raw data(w/o background correction)
Average(w/o background correction)
For 1E3 ≤ copy/rxn ≤ 1E8,
n_Emax = -3.51765 log[copy/rxn] + 41.35109 (R²=0.9995)
Efficiency = 10(-1/slope) - 1 = 0.9243
For 1E1 ≤ copy/rxn ≤ 1E8,
n_Emax = -2.94369 log[copy/rxn] + 37.78416 (R²=0.9689)
Efficiency = 10(-1/slope) - 1 = 1.186297
For 1E3 ≤ copy/rxn ≤ 1E8,
n_Emax = -3.53953 log[copy/rxn] + 37.52614 (R²=0.9987)
Efficiency = 10(-1/slope) - 1 = 0.9166
For 1E0 ≤ copy/rxn ≤ 1E8,
n_Emax = -2.80534 log[copy/rxn] + 32.9833 (R²=0.9511)
Efficiency = 10(-1/slope) - 1 = 1.2722
n_Emax
50
45
40
35
30
25
20
15
10
5
0
1.0E+00
1.0E+01
1.0E+02
1.0E+03
1.0E+04
1.0E+05
1.0E+06
1.0E+07
1.0E+08
log(copy/reaction)

FIG. 16

WITHOUT BACKGROUND FLUORESCENCE INTENSITY CORRECTION

| copy/rxn | Average(nEmax) | St.Dev.(nEmax) | %CV of nEmax |
|---|---|---|---|
| 1.00E+08 | 3.458E+00 | 3.314E-01 | 9.58% |
| 1.00E+07 | 1.183E+01 | 7.115E-01 | 6.02% |
| 1.00E+06 | 1.502E+01 | 1.040E+00 | 6.92% |
| 1.00E+05 | 1.896E+01 | 1.740E+00 | 9.18% |
| 1.00E+04 | 2.152E+01 | 1.611E+00 | 7.49% |
| 1.00E+03 | 2.556E+01 | 3.020E+00 | 11.82% |
| 1.00E+02 | 3.225E+01 | 6.556E+00 | 20.33% |
| 1.00E+01 | 3.101E+01 | 5.792E+00 | 18.68% |
| 1.00E+00 | 3.484E+01 | 1.325E+01 | 38.03% |

WITHOUT BACKGROUND FLUORESCENCE INTENSITY CORRECTION

| copy/rxn | Average(nEmax) | St.Dev.(nEmax) | %CV of nEmax |
|---|---|---|---|
| 1.00E+08 | 1.32E+01 | 1.576E-01 | 1.19% |
| 1.00E+07 | 1.655E+01 | 1.381E-01 | 0.83% |
| 1.00E+06 | 2.040E+01 | 2.441E-01 | 1.20% |
| 1.00E+05 | 2.412E+01 | 2.726E-01 | 1.13% |
| 1.00E+04 | 2.770E+01 | 2.899E-01 | 1.08% |
| 1.00E+03 | 3.164E+01 | 1.246E+00 | 3.94% |
| 1.00E+05 | 3.435E+01 | 7.591E+00 | 22.10% |
| 1.00E+04 | 3.821E+01 | 6.954E+00 | 18.20% |
| 1.00E+03 | 3.033E+01 | 1.747E+01 | 57.60% |

FIG. 17

for 1E3 ≤ copy/rxn ≤ 1E8,

$n_{Emax}$ = -3.51765 log[copy/rxn] + 41.35109 ($R^2$=0.9995)

$n_{Emax}$ = 20.25 for 1E6 copy/rxn

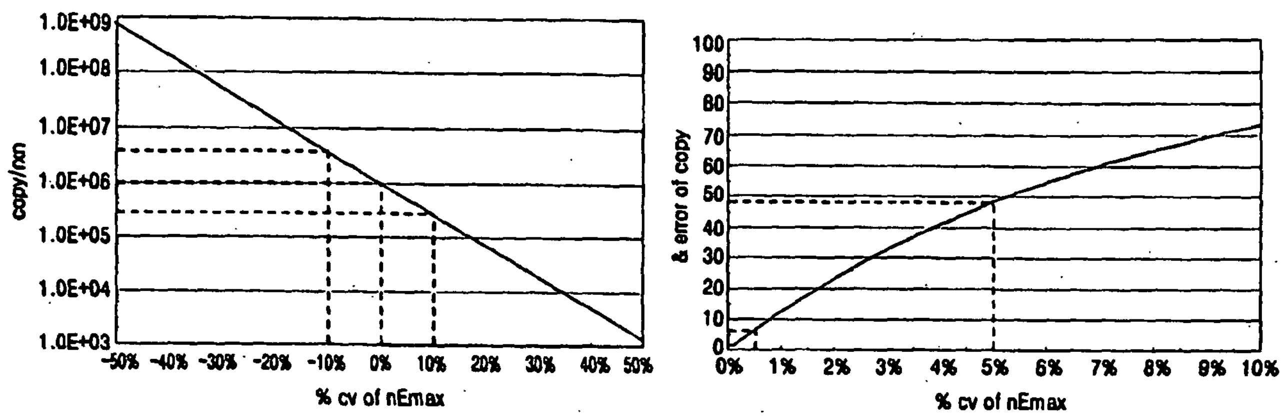


$$\text{error in copy} = \frac{(copy)_{cal} - (copy)_{exp}}{(copy)_{exp}}$$

$$\text{error in log[copy]} = \frac{\log(copy)_{cal} - \log(copy)_{exp}}{\log(copy)_{exp}}$$

0.5% cv of $n_{Emax}$= 6.85% error in copy=0.48% error in log[copy]

5.0% cv of $n_{Emax}$= 48.5% error in copy=4.80% error in log[copy]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A
- US 4683202 A
- US 6303305 B
- US 6503720 B
- US 20030165832 A
- US 20020031768 A